# EUROPÄISCHE PATENTSCHRIFT

(11) **EP 1 272 645 B1**
(45) Veröffentlichungstag und Bekanntmachung des Hinweises auf die Patenterteilung: **25.08.2004**
(21) Anmeldenummer: 01907531.6
(22) Anmeldetag: 14.02.2001
(51) Int. Cl.: C12N 15/61, C12N 15/82, C12N 9/90, C12N 5/10, C12P 19/24, A01H 5/00

(54) **PRODUKTION NICHT-KARIOGENER ZUCKER IN TRANSGENEN PFLANZEN**
PRODUCTION OF NON-CARIOGENIC SUGARS IN TRANSGENIC PLANTS
PRODUCTION DE SUCRES NON CARIOGENES DANS DES PLANTES TRANSGENIQUES

(30) Priorität: 14.02.2000 DE 10006462
(43) Veröffentlichungstag der Anmeldung: 08.01.2003
(73) Patentinhaber: IPK Institut für Pflanzengenetik und Kulturpflanzenforschung, 06466 Gatersleben (DE)
(72) Erfinder: BÖRNKE, Frederik, 06484 Quedlinburg (DE); SONNEWALD, Uwe, 06484 Quedlinburg (DE)
(74) Vertreter: Neuefeind, Regina
(86) Internationale Anmeldenummer: PCT/EP2001/001603
(87) Internationale Veröffentlichungsnummer: WO 2001/059136

(56) Entgegenhaltungen:
- EP-A- 0 113 592
- WO-A-95/20047
- DATABASE WPI Section Ch, Week 199423 Derwent Publications Ltd., London, GB; Class C12,Page 4, AN 1990-357860 XP002168569 & JP 06 046951 B (NISSHIN SEITO), 1994

### SEQUENZPROTOKOLL

<110> IPK - Institut für Pflanzengenetik und Kulturpflan
<120> Produktion nicht-kariogener Zucker in transgenen Pflanzen
<130> I 7212
<140>
   <141>
<150> DE 100 06 462.0
   <151> 2000-02-14
<160> 3
<170> PatentIn Ver. 2.1
<210> 1
   <211> 1803
   <212> DNA
   <213> Erwinia rhapontici
<400> 1
   atgtcctctc aaggattgaa aacggctgtc gctatttttc ttgcaaccac tttttctgcc 60
   acatcctatc aggcctgcag tgccgggcca gataccgccc cctcactcac cgttcagcaa 120
   tcaaatgccc tgcccacatg gtggaagcag gctgtttttt atcaggtata tccacgctca 180
   tttaaagata cgaatgggga tggcattggg gatttaaacg gtattattga gaatttagac 240
   tatctgaaga aactgggtat tgatgcgatt tggatcaatc cacattacga ttcgccgaat 300
   acggataatg gttatgacat ccgggattac cgtaagataa tgaaagaata cggtacgatg 360
   gaagactttg accgtcttat ttcagaaatg aagaaacgca atatgcgttt gatgattgat 420
   attgttatca accacaccag cgatcagcat gcctggtttg ttcagagcaa atcgggtaag 480
   aacaacccct acagggacta ttacttctgg cgtgacggta aggatggcca tgcccccaat 540
   aactatccct ccttcttcgg tggctcagcc tgggaaaaag acgataaatc aggccagtat 600
   tacctccatt actttgccaa acagcaaccc gacctcaact gggacaatcc caaagtccgt 660
   caagacctgt atgacatgct ccgcttctgg ttagataaag gcgtttctgg tttacgcttt 720
   gataccgttg ccacctactc gaaaatcccg aacttccctg accttagcca acagcagtta 780
   aaaaatttcg ccgaggaata tactaaaggt cctaaaattc acgactacgt gaatgaaatg 840
   aacagagaag tattatccca ctatgatatc gccactgcgg gggaaatatt tggggttcct 900
   ctggataaat cgattaagtt tttcgatcgc cgtagaaatg aattaaatat agcgtttacg 960
   tttgatctga tcaggctcga tcgtgatgct gatgaaagat ggcggcgaaa agactggacc 1020
   ctttcgcagt tccgaaaaat tgtcgataag gttgaccaaa cggcaggaga gtatgggtgg 1080
   aatgcctttt tcttagacaa tcacgacaat ccccgcgcgg tttctcactt tggtgatgat 1140
   cgaccacaat ggcgcgagca tgcggcgaaa gcactggcaa cattgacgct gacccagcgt 1200
   gcaacgccgt ttatctatca gggttcagaa ctcggtatga ccaattatcc ctttaaaaaa 1260
   atcgatgatt tcgatgatgt agaggtgaaa ggtttttggc aagactacgt tgaaacaggc 1320
   aaagtgaaag ctgaggaatt ccttcaaaac gtacgccaaa ccagccgtga taacagcaga 1380
   acccccttcc agtgggatgc aagcaaaaac gcgggcttta ccagtggaac cccctggtta 1440
   aaaatcaatc ccaattataa agaaatcaac agcgcagatc agattaataa tccaaattcc 1500
   gtatttaact attatagaaa gctgattaac attcgccatg acatccctgc cttgacctac 1560
   ggcagttata ttgatttaga ccctgacaac aattcagtct atgcttacac ccgaacgctc 1620
   ggcgctgaaa aatatcttgt ggtcattaat tttaaagaag aagtgatgca ctacaccctg 1680
   cccggggatt tatccatcaa taaggtgatt actgaaaaca acagtcacac tattgtgaat 1740
   aaaaatgaca ggcaactccg tcttgaaccc tggcagtcgg gcatttataa acttaatccg 1800
   tag                                                               1803
<210> 2
   <211> 1803
   <212> DNA
   <213> Erwinia rhapontici
<220>
   <221> CDS
   <222> (1)..(1800)
<400> 2
   atg tcc tct caa gga ttg aaa acg gct gtc gct att ttt ctt gca acc    48
   Met Ser Ser Gln Gly Leu Lys Thr Ala Val Ala Ile Phe Leu Ala Thr
     1               5                  10                  15
   act ttt tct gcc aca tcc tat cag gcc tgc agt gcc ggg cca gat acc    96
   Thr Phe Ser Ala Thr Ser Tyr Gln Ala Cys Ser Ala Gly Pro Asp Thr
                20                  25                  30
   gcc ccc tca ctc acc gtt cag caa tca aat gcc ctg ccc aca tgg tgg    144
   Ala Pro Ser Leu Thr Val Gln Gln Ser Asn Ala Leu Pro Thr Trp Trp
            35                  40                  45
   aag cag gct gtt ttt tat cag gta tat cca cgc tca ttt aaa gat acg    192
   Lys Gln Ala Val Phe Tyr Gln Val Tyr Pro Arg Ser Phe Lys Asp Thr
        50                  55                  60
   aat ggg gat ggc att ggg gat tta aac ggt att att gag aat tta gac    240
   Asn Gly Asp Gly Ile Gly Asp Leu Asn Gly Ile Ile Glu Asn Leu Asp
    65                  70                  75                  80
   tat ctg aag aaa ctg ggt att gat gcg att tgg atc aat cca cat tac    288
   Tyr Leu Lys Lys Leu Gly Ile Asp Ala Ile Trp Ile Asn Pro His Tyr
                    85                  90                  95
   gat tcg ccg aat acg gat aat ggt tat gac atc cgg gat tac cgt aag    336
   Asp Ser Pro Asn Thr Asp Asn Gly Tyr Asp Ile Arg Asp Tyr Arg Lys
               100                 105                 110
   ata atg aaa gaa tac ggt acg atg gaa gac ttt gac cgt ctt att tca    384
   Ile Met Lys Glu Tyr Gly Thr Met Glu Asp Phe Asp Arg Leu Ile Ser
           115                 120                 125
   gaa atg aag aaa cgc aat atg cgt ttg atg att gat att gtt atc aac    432
   Glu Met Lys Lys Arg Asn Met Arg Leu Met Ile Asp Ile Val Ile Asn
       130                 135                 140
   cac acc agc gat cag cat gcc tgg ttt gtt cag agc aaa tcg ggt aag    480
   His Thr Ser Asp Gln His Ala Trp Phe Val Gln Ser Lys Ser Gly Lys
   145                 150                 155                 160
   aac aac ccc tac agg gac tat tac ttc tgg cgt gac ggt aag gat ggc    528
   Asn Asn Pro Tyr Arg Asp Tyr Tyr Phe Trp Arg Asp Gly Lys Asp Gly
                   165                 170                 175
   cat gcc ccc aat aac tat ccc tcc ttc ttc ggt ggc tca gcc tgg gaa    576
   His Ala Pro Asn Asn Tyr Pro Ser Phe Phe Gly Gly Ser Ala Trp Glu
               180                 185                 190
   aaa gac gat aaa tca ggc cag tat tac ctc cat tac ttt gcc aaa cag    624
   Lys Asp Asp Lys Ser Gly Gln Tyr Tyr Leu His Tyr Phe Ala Lys Gln
           195                 200                 205
   caa ccc gac ctc aac tgg gac aat ccc aaa gtc cgt caa gac ctg tat    672
   Gln Pro Asp Leu Asn Trp Asp Asn Pro Lys Val Arg Gln Asp Leu Tyr
       210                 215                 220
   gac atg ctc cgc ttc tgg tta gat aaa ggc gtt tct ggt tta cgc ttt    720
   Asp Met Leu Arg Phe Trp Leu Asp Lys Gly Val Ser Gly Leu Arg Phe
   225                 230                 235                 240
   gat acc gtt gcc acc tac tcg aaa atc ccg aac ttc cct gac ctt agc    768
   Asp Thr Val Ala Thr Tyr Ser Lys Ile Pro Asn Phe Pro Asp Leu Ser
                   245                 250                 255
   caa cag cag tta aaa aat ttc gcc gag gaa tat act aaa ggt cct aaa    816
   Gln Gln Gln Leu Lys Asn Phe Ala Glu Glu Tyr Thr Lys Gly Pro Lys
               260                 265                 270
   att cac gac tac gtg aat gaa atg aac aga gaa gta tta tcc cac tat    864
   Ile His Asp Tyr Val Asn Glu Met Asn Arg Glu Val Leu Ser His Tyr
           275                 280                 285
   gat atc gcc act gcg ggg gaa ata ttt ggg gtt cct ctg gat aaa tcg    912
   Asp Ile Ala Thr Ala Gly Glu Ile Phe Gly Val Pro Leu Asp Lys Ser
       290                 295                 300
   att aag ttt ttc gat cgc cgt aga aat gaa tta aat ata gcg ttt acg    960
   Ile Lys Phe Phe Asp Arg Arg Arg Asn Glu Leu Asn Ile Ala Phe Thr
   305                 310                 315                 320
   ttt gat ctg atc agg ctc gat cgt gat gct gat gaa aga tgg cgg cga   1008
   Phe Asp Leu Ile Arg Leu Asp Arg Asp Ala Asp Glu Arg Trp Arg Arg
                   325                 330                 335
   aaa gac tgg acc ctt tcg cag ttc cga aaa att gtc gat aag gtt gac   1056
   Lys Asp Trp Thr Leu Ser Gln Phe Arg Lys Ile Val Asp Lys Val Asp
               340                 345                 350
   caa acg gca gga gag tat ggg tgg aat gcc ttt ttc tta gac aat cac   1104
   Gln Thr Ala Gly Glu Tyr Gly Trp Asn Ala Phe Phe Leu Asp Asn His
           355                 360                 365
   gac aat ccc cgc gcg gtt tct cac ttt ggt gat gat cga cca caa tgg   1152
   Asp Asn Pro Arg Ala Val Ser His Phe Gly Asp Asp Arg Pro Gln Trp
       370                 375                 380
   cgc gag cat gcg gcg aaa gca ctg gca aca ttg acg ctg acc cag cgt   1200
   Arg Glu His Ala Ala Lys Ala Leu Ala Thr Leu Thr Leu Thr Gln Arg
   385                 390                 395                 400
   gca acg ccg ttt atc tat cag ggt tca gaa ctc ggt atg acc aat tat   1248
   Ala Thr Pro Phe Ile Tyr Gln Gly Ser Glu Leu Gly Met Thr Asn Tyr
                   405                 410                 415
   ccc ttt aaa aaa atc gat gat ttc gat gat gta gag gtg aaa ggt ttt   1296
   Pro Phe Lys Lys Ile Asp Asp Phe Asp Asp Val Glu Val Lys Gly Phe
               420                 425                 430
   tgg caa gac tac gtt gaa aca ggc aaa gtg aaa gct gag gaa ttc ctt   1344
   Trp Gln Asp Tyr Val Glu Thr Gly Lys Val Lys Ala Glu Glu Phe Leu
           435                 440                 445
   caa aac gta cgc caa acc agc cgt gat aac agc aga acc ccc ttc cag   1392
   Gln Asn Val Arg Gln Thr Ser Arg Asp Asn Ser Arg Thr Pro Phe Gln
       450                 455                 460
   tgg gat gca agc aaa aac gcg ggc ttt acc agt gga acc ccc tgg tta   1440
   Trp Asp Ala Ser Lys Asn Ala Gly Phe Thr Ser Gly Thr Pro Trp Leu
   465                 470                 475                 480
   aaa atc aat ccc aat tat aaa gaa atc aac agc gca gat cag att aat   1488
   Lys Ile Asn Pro Asn Tyr Lys Glu Ile Asn Ser Ala Asp Gln Ile Asn
                   485                 490                 495
   aat cca aat tcc gta ttt aac tat tat aga aag ctg att aac att cgc     1536
   Asn Pro Asn Ser Val Phe Asn Tyr Tyr Arg Lys Leu Ile Asn Ile Arg
               500                 505                 510
   cat gac atc cct gcc ttg acc tac ggc agt tat att gat tta gac cct     1584
   His Asp Ile Pro Ala Leu Thr Tyr Gly Ser Tyr Ile Asp Leu Asp Pro
           515                 520                 525
   gac aac aat tca gtc tat gct tac acc cga acg ctc ggc gct gaa aaa     1632
   Asp Asn Asn Ser Val Tyr Ala Tyr Thr Arg Thr Leu Gly Ala Glu Lys
       530                 535                 540
   tat ctt gtg gtc att aat ttt aaa gaa gaa gtg atg cac tac acc ctg     1680
   Tyr Leu Val Val Ile Asn Phe Lys Glu Glu Val Met His Tyr Thr Leu
   545                 550                 555                 560
   ccc ggg gat tta tcc atc aat aag gtg att act gaa aac aac agt cac     1728
   Pro Gly Asp Leu Ser Ile Asn Lys Val Ile Thr Glu Asn Asn Ser His
                   565                 570                 575
   act att gtg aat aaa aat gac agg caa ctc cgt ctt gaa ccc tgg cag     1776
   Thr Ile Val Asn Lys Asn Asp Arg Gln Leu Arg Leu Glu Pro Trp Gln
               580                 585                 590
   tcg ggc att tat aaa ctt aat ccg tag                                 1803
   Ser Gly Ile Tyr Lys Leu Asn Pro
           595                 600
<210> 3
   <211> 600
   <212> PRT
   <213> Erwinia rhapontici
<400> 3
   Met Ser Ser Gln Gly Leu Lys Thr Ala Val Ala Ile Phe Leu Ala Thr
   1 5 10 15
   Thr Phe Ser Ala Thr Ser Tyr Gln Ala Cys Ser Ala Gly Pro Asp Thr
   20 25 30
   Ala Pro Ser Leu Thr Val Gln Gln Ser Asn Ala Leu Pro Thr Trp Trp
   35 40 45
   Lys Gln Ala Val Phe Tyr Gln Val Tyr Pro Arg Ser Phe Lys Asp Thr
   50 55 60
   Asn Gly Asp Gly Ile Gly Asp Leu Asn Gly Ile Ile Glu Asn Leu Asp
   65 70 75 80
   Tyr Leu Lys Lys Leu Gly Ile Asp Ala Ile Trp Ile Asn Pro His Tyr
   85 90 95
   Asp Ser Pro Asn Thr Asp Asn Gly Tyr Asp Ile Arg Asp Tyr Arg Lys
   100 105 110
   Ile Met Lys Glu Tyr Gly Thr Met Glu Asp Phe Asp Arg Leu Ile Ser
   115 120 125
   Glu Met Lys Lys Arg Asn Met Arg Leu Met Ile Asp Ile Val Ile Asn
   130 135 140
   His Thr Ser Asp Gln His Ala Trp Phe Val Gln Ser Lys Ser Gly Lys
   145 150 155 160
   Asn Asn Pro Tyr Arg Asp Tyr Tyr Phe Trp Arg Asp Gly Lys Asp Gly
   165 170 175
   His Ala Pro Asn Asn Tyr Pro Ser Phe Phe Gly Gly Ser Ala Trp Glu
   180 185 190
   Lys Asp Asp Lys Ser Gly Gln Tyr Tyr Leu His Tyr Phe Ala Lys Gln
   195 200 205
   Gln Pro Asp Leu Asn Trp Asp Asn Pro Lys Val Arg Gln Asp Leu Tyr
   210 215 220
   Asp Met Leu Arg Phe Trp Leu Asp Lys Gly Val Ser Gly Leu Arg Phe
   225 230 235 240
   Asp Thr Val Ala Thr Tyr Ser Lys Ile Pro Asn Phe Pro Asp Leu Ser
   245 250 255
   Gln Gln Gln Leu Lys Asn Phe Ala Glu Glu Tyr Thr Lys Gly Pro Lys
   260 265 270
   Ile His Asp Tyr Val Asn Glu Met Asn Arg Glu Val Leu Ser His Tyr
   275 280 285
   Asp Ile Ala Thr Ala Gly Glu Ile Phe Gly Val Pro Leu Asp Lys Ser
   290 295 300
   Ile Lys Phe Phe Asp Arg Arg Arg Asn Glu Leu Asn Ile Ala Phe Thr
   305 310 315 320
   Phe Asp Leu Ile Arg Leu Asp Arg Asp Ala Asp Glu Arg Trp Arg Arg
   325 330 335
   Lys Asp Trp Thr Leu Ser Gln Phe Arg Lys Ile Val Asp Lys Val Asp
   340 345 350
   Gln Thr Ala Gly Glu Tyr Gly Trp Asn Ala Phe Phe Leu Asp Asn His
   355 360 365
   Asp Asn Pro Arg Ala Val Ser His Phe Gly Asp Asp Arg Pro Gln Trp
   370 375 380
   Arg Glu His Ala Ala Lys Ala Leu Ala Thr Leu Thr Leu Thr Gln Arg
   385 390 395 400
   Ala Thr Pro Phe Ile Tyr Gln Gly Ser Glu Leu Gly Met Thr Asn Tyr
   405 410 415
   Pro Phe Lys Lys Ile Asp Asp Phe Asp Asp Val Glu Val Lys Gly Phe
   420 425 430
   Trp Gln Asp Tyr Val Glu Thr Gly Lys Val Lys Ala Glu Glu Phe Leu
   435 440 445
   Gln Asn Val Arg Gln Thr Ser Arg Asp Asn Ser Arg Thr Pro Phe Gln
   450 455 460
   Trp Asp Ala Ser Lys Asn Ala Gly Phe Thr Ser Gly Thr Pro Trp Leu
   465 470 475 480
   Lys Ile Asn Pro Asn Tyr Lys Glu Ile Asn Ser Ala Asp Gln Ile Asn
   485 490 495
   Asn Pro Asn Ser Val Phe Asn Tyr Tyr Arg Lys Leu Ile Asn Ile Arg
   500 505 510
   His Asp Ile Pro Ala Leu Thr Tyr Gly Ser Tyr Ile Asp Leu Asp Pro
   515 520 525
   Asp Asn Asn Ser Val Tyr Ala Tyr Thr Arg Thr Leu Gly Ala Glu Lys
   530 535 540
   Tyr Leu Val Val Ile Asn Phe Lys Glu Glu Val Met His Tyr Thr Leu
   545 550 555 560
   Pro Gly Asp Leu Ser Ile Asn Lys Val Ile Thr Glu Asn Asn Ser His
   565 570 575
   Thr Ile Val Asn Lys Asn Asp Arg Gln Leu Arg Leu Glu Pro Trp Gln
   580 585 590
   Ser Gly Ile Tyr Lys Leu Asn Pro
   595 600

## Beschreibung

Die vorliegende Erfindung betrifft rekombinante Nukleinsäuremolelüle, die eine DNA-Sequenz enthalten, die ein Protein mit der enzymatischen Aktivität einer Saccharoseisomerase kodiert, und worin diese DNA-Sequenz unter Kontrolle von regulatorischen Sequenzen eines in Pflanzen aktiven Promotors steht. Weiter betrifft die Erfindung Vektoren und Wirtszellen, die die erfindungsgemäßen rekombinanten Nukleinsäuremoleküle enthalten. Weiter werden Verfahren zur Herstellung transformierter Pflanzenzellen und Pflanzen unter Verwendung der rekombinanten Nukleinsäuremoleküle und Vektoren bereitgestellt. Weiter betrifft die Erfindung transgene Pflanzen, deren Ernteprodukte und Vermehrungsmaterial sowie transgene Pflanzenzellen, die die erfindungsgemäßen Nukleinsäuremoleküle oder Vektoren enthalten oder durch das erfindungsgemäße Verfahren hergestellt werden. Die Erfindung betrifft insbesondere auch ein Verfahren zur Produktion nicht-kariogener Zucker in Pflanzenzellen und Pflanzen, umfassend die Übertragung eines erfindungsgemäßen Nukleinsäuremoleküls oder Vektors auf Pflanzenzellen. Weiter betrifft die Erfindung die Verwendung von DNA-Sequenzen, die ein Protein mit der enzymatischen Aktivität einer Saccharoseisomerase kodieren, zur Produktion nicht-kariogener Zucker in Pflanzenzellen und Pflanzen.

Die nicht-kariogenen Zuckerersatzstoffe bzw. Süßstoffe Palatinose (Isomaltulose) und Trehalulose werden großtechnisch aus Saccharose durch eine enzymatische Umlagerung unter Verwendung von immobilisierten Bakterienzellen hergestellt. Dabei wird die zwischen den Monosacchariden des Disaccharids Saccharose bestehende α1→β2-glykosidische Bindung zu einer α1→6-Bindung bei Palatinose bzw. einer α1→α1-Bindung bei Trehalulose isomerisiert. Diese Umlagerung von Saccharose zu den beiden nicht-kariogenen Disacchariden erfolgt unter Katalyse des bakteriellen Enzyms Saccharoseisomerase, auch Saccharosemutase genannt. Je nach verwendetem Mikroorganismus ergibt sich bei der Reaktion ein Reaktionsgemisch, das neben den erwünschten nicht-kariogenen Disacchariden Palatinose und Trehalulose auch gewisse Anteile an unerwünschten Monosacchariden, Glukose und/oder Fruktose, enthält. Diese Monosaccharidanteile sind ein erhebliches technisches Problem, da zu ihrer Abtrennung aufwendige Reinigungsprozeduren erforderlich sind. Die Saccharose wird nach den Verfahren des Standes der Technik entweder aus Zuckerrohr oder Zuckerrübe durch Kristallisation gereinigt.

Bei der Produktion von nicht-kariogenen Disacchariden können Monosaccharide einerseits durch Abbau der gewünschten Endprodukte Palatinose und/oder Trehalulose und andererseits durch Abbau des Ausgangsprodukts Saccharose erzeugt werden. Um den Anteil an unerwünschten Monosacchariden zu verringern, werden in PCT/EP95/00165 Bakterienzellen vorgeschlagen, die einen reduzierten Palatinoseund/oder Trehalulose-Stoffwechsel aufweisen, also diese Disaccharide nur in geringem Umfang metabolisch verwerten können. Mit dem gleichen Ziel werden in EP-A2-0 751 218 Bakterienzellen offenbart, die einen reduzierten Saccharose-Stoffwechsel aufweisen. Transgene Pflanzenzellen und Pflanzen, die in der Lage sind, nicht-kariogene Zucker zu synthetisieren, die natürlicherweise nicht in Pflanzen vorkommen, werden im Stand der Technik nicht beschrieben.

Der vorliegenden Erfindung liegt somit die Aufgabe zugrunde, ein neues Verfahren zur Produktion nicht-kariogener Zucker bereitzustellen, das die Nachteile des Standes der Technik überwindet und insbesondere ohne aufwendige Veredelungsschritte auskommt.

Diese und weitere Aufgaben werden durch die in den Patentansprüchen definierten Ausführungsformen gelöst.

Es wurde jetzt überraschenderweise gefunden, dass nicht-kariogene Zucker, insbesondere Palatinose und Trehalulose, in transformierten Pflanzenzellen und Pflanzen produziert werden können, in die eine für eine Saccharoseisomerase kodierende DNA-Sequenz gentechnisch eingeführt wurde und in denen ein Protein mit der enzymatischen Aktivität einer Saccharoseisomerase synthetisiert wird. Dies bedeutet, dass transgene Pflanzenzellen und Pflanzen auf einfache und effiziente Weise als Produktionsstätte für nicht-kariogene Zucker genutzt werden können.

Wie erwähnt, katalysieren Proteine mit Saccharoseisomerase-Aktivität die Isomerisierung des Disaccharids Saccharose zu anderen Disacchariden, wie Palatinose und Trehalulose. Beispiele für Organismen, deren Zellen ein Protein mit Saccharoseisomerase-Aktivität kodierende Nukleinsäuresequenzen enthalten, sind insbesondere Mikroorganismen der Gattungen Protaminobacter, Erwinia, Serratia, Leuconostoc, Pseudomonas, Agrobacterium, Klebsiella und Enterobacter. Insbesondere sind hier folgende Beispiele für solche Mikroorgansimen zu nennen: *Protaminobacter rubrum* (CBS 547, 77), *Erwinia rhapontici* (NCPPB 1578), *Serratia plymuthica* (ATCC 15928), *Serratia marcescens* (NCIB 8285), *Leuconostoc mesenteroides* NRRL B-521f (ATCC 10830a). *Pseudomonas mesoacidophila* MX-45 (FERM 11808 bzw. FERM BP 3619), *Agrobacterium radiobacter* MX-232 (FERM 12397 bzw. FERM BP 3620), *Klebsiella subspezies* und *Enterobacter spezies.*

Auch DNA-Sequenzen, die Proteine mit Saccharoseisomerase-Aktivität kodieren, sind im Stand der Technik bekannt und stehen dem Fachmann somit für den Transfer auf Pflanzenzellen zur Verfügung. So sind bspw. solche Sequenzen aus *Protaminobacler rubrum, Erwinia rhapontici. Enterobacter species* SZ 62 und *Pseudomonas mesoacidophila* MX-45 in PCT/EP 95/00165 beschrieben. Auf die Offenbarung dieser Patentanmeldung wird hiermit Bezug genommen, sowohl hinsichtlich der offenbarten Sequenzen selbst, als auch im Hinblick auf die Auffindung und Charakterisierung dieser und weiterer Saccharoseisomerase kodierender Sequenzen aus anderen Quellen.

Weitere Saccharoseisomerase kodierende DNA-Sequenzen kann der Fachmann u.a. den Gendatenbanken unter Verwendung geeigneter Suchprofile und Computerprogramme für das Screenen nach homologen Sequenzen bzw. für Sequenzvergleiche entnehmen.

Darüber hinaus kann der Fachmann weitere Saccharoseisomerase kodierende DNA-Sequenzen aus anderen Organismen mittels herkömmlicher molekularbiologischer Techniken selbst auffinden und im Rahmen der vorliegenden Erfindung einsetzen. So kann der Fachmann bspw. geeignete Hybridisierungssonden von den bekannten Saccharoseisomerase-Sequenzen ableiten und für das Screening von cDNAund/oder genomischen Banken des jeweils gewünschten Organismus, aus dem ein neues Saccharoseisomerase-Gen isoliert werden soll, einsetzen. Hierbei kann der Fachmann auf geläufige Hybridisierungs-, Klonierungs- und Sequenzierungsmethoden zurückgreifen, die in jedem bio- oder gentechnologischen Labor wohl bekannt und etabliert sind (siehe z.B. Sambrook *et al.* (1989) Molecular Cloning: A Laboratory Manual, 2. Auflage, Cold Spring Harbor Laboratory Press, Cold Spring Harbor, New York). Ebenso ist der Fachmann natürlich in der Lage, anhand bekannter Saccharoseisomerase-DNA-Sequenzen geeignete Oligonukleotide als Primer für Klonierungen neuer Gene mittels PCR zu synthetisieren und erfolgreich einzusetzen.

Die Erfindung betrifft somit ein rekombinantes Nukleinsäuremolekül, umfassend
a) regulatorische Sequenzen eines in Pflanzenzellen aktiven Promotors;
b) operativ daran gebunden eine DNA-Sequenz. die ein Protein mit der enzymatischen Aktivität einer Saccharoseisomerase kodiert; und
c) operativ daran gebunden regulatorische Sequenzen, die als Transkriptions-, Terminations- und/oder Polyadenylierungssignale in Pflanzenzellen dienen können.

Im Zusammenhang mit der vorliegenden Erfindung wird unter einem Protein mit der enzymatischen Aktivität einer Saccharoseisomerase ein Protein verstanden, das die Isomerisierung von Saccharose zu anderen Disacchariden katalysiert, wobei die α1 → β2-glykosidische Bindung zwischen Glukose und Fruktose in der Saccharose in eine andere glykosidische Bindung zwischen zwei Monosaccharideinheiten überführt wird, insbesondere in eine α1 →6-Bindung und/oder α1→α1 -Bindung. Besonders bevorzugt wird unter einem Protein mit der enzymatischen Aktivität einer Saccharoseisomerase ein Protein verstanden, das zur Isomerisierung von Saccharose zu Palatinose und/oder Trehalulose befähigt ist. Dabei beträgt der Anteil von Palatinose und Trehalulose an den gesamten Disacchariden, die durch Isomerisierung von Saccharose gebildet werden ≥ 2%, bevorzugt ≥ 20%, besonders bevorzugt ≥ 50% und am meisten bevorzugt ≥ 60%.

Die DNA-Sequenz, die ein Protein mit der enzymatischen Aktivität einer Saccharoseisomerase kodiert, kann aus natürlichen Quellen isoliert oder nach herkömmlichen Verfahren synthetisiert werden. Mittels gängiger molekularbiologischer Techniken (siehe beispielsweise Sambrook *et al.* (1989) Molecular Cloning: A Laboratory Manual, 2. Auflage, Cold Spring Harbor Laboratory Press, Cold Spring Harbor, New York) ist es möglich, gewünschte Konstrukte für die Transformation von Pflanzenzellen vorzubereiten bzw. herzustellen. Die für die gentechnische Manipulation in prokaryontischen Zellen üblicherweise eingesetzten Klonierungs-, Mutaeenisierungs-. Sequenzanalyse-, Restriktionsanalyse- und weitere biochemisch-molekularbiologischen Methoden sind dem Durchschnittsfachmann wohl bekannt. So können nicht nur geeignete chimäre Genkonstrukte mit der gewünschten Fusion von Promotor und Saccharoseisomerase-DNA-Sequenz und ggf. weiteren Regulations- und/oder Signalsequenzen hergestellt werden, vielmehr kann der Fachmann, falls erwünscht, zusätzlich mittels Routinetechniken, verschiedenartige Mutationen in die die Saccharoseisomerase kodierende DNA-Sequenz einführen, wodurch es zur Synthese von Proteinen mit eventuell veränderten biologischen Eigenschaften kommt. Hierbei ist zum einen die Erzeugung von Deletionsmutanten möglich, bei denen durch fortschreitende Deletion vom 5'- oder vom 3'-Ende der kodierenden DNA-Sequenz die Synthese entsprechend verkürzter Proteine erreicht werden kann. Ferner ist es möglich, gezielt Enzyme herzustellen, die durch Addition entsprechender Signalsequenzen in bestimmten Kompartimenten der Pflanzenzelle lokalisiert sind. Derartige Sequenzen sind in der Literatur beschrieben und dem Durchschnittsfachmann wohl bekannt (siehe z.B. Braun *et al.* (1992) EMBO J. 11:3219-3227; Wolter F. *et al.* (1988) Proc. Natl. Acad. Sci. USA 85:846-850; Sonnewald U. *et al.* (1991) Plant J. 1:95-106). Weiterhin ist auch die Einführung von Punktmutationen an Positionen denkbar, bei denen eine Veränderung der Aminosäuresequenz einen Einfluss beispielsweise auf die Enzymaktivität oder die Regulierung des Enzyms hat. Auf diese Weise können z.B. Mutanten hergestellt werden, die nicht mehr den normalerweise in der Zelle herrschenden Regulationsmechanismen über allosterische Regulation oder kovalente Modifizierung unterliegen. Des weiteren können Mutanten hergestellt werden, die eine veränderte Substrat- oder Produktspezifität aufweisen. Weiterhin können Mutanten hergestellt werden, die ein verändertes Aktivitäts-, Temperatur- und/oder pH-Profil aufweisen.

Für die gentechnische Manipulation in prokaryontischen Zellen können die erfindungsgemäßen rekombinanten Nukleinsäuremoleküle oder Teile davon in Plasmide eingebracht werden, die eine Mutagenese oder eine Sequenzveränderung durch Rekombination von DNA-Sequenzen erlauben. Mit Hilfe von Standardverfahren (siehe z.B. Sambrook *et al.* (1989), vide supra) können Basenaustausche vorgenommen oder natürliche oder synthetische Sequenzen hinzugefügt werden. Für die Verbindung der DNA-Fragmente untereinander können an die Fragmente, wo erforderlich, Adapter oder Linker angefügt werden. Ferner können mittels enzymatischer und anderer Manipulationen passende Restriktionsschnittstellen zur Verfügung gestellt oder überflüssige DNA oder Restriktionsschnittstellen entfernt werden. Wo Insertionen, Deletionen oder Substitutionen in Frage kommen, können in vitro-Mutagenese, "primer repair", Restriktion oder Ligation verwendet werden. Als Analysemethoden werden im allgemeinen Sequenzanalyse, Restriktionsanalyse und weitere biochemischmolekularbiologische Methoden durchgeführt.

In einer bevorzugten Ausführungsform ist die DNA-Sequenz, die ein Protein mit der enzymatischen Aktivität einer Saccharoseisomerase kodiert, ausgewählt aus der Gruppe bestehend aus
a) DNA-Sequenzen, die eine Nukleotidsequenz umfassen, die die in SEQ ID NO. 3 angegebene Aminosäuresequenz oder Fragmente davon kodieren,
b) DNA-Sequenzen, die die in SEQ ID No. 1 angegebene Nukleotidsequenz oder Teile davon kodieren,
c) DNA-Sequenzen, die eine Nukleotidsequenz, die mit einem komplementären Strang der Nukleotidsequenz von a) oder b) hybridisieren, oder Teile dieser Nukleotidsequenz umfassen,
d) DNA-Sequenzen, die eine Nukleotidsequenz, die zu einer Nukleotidsequenz von c) degeneriert ist, oder Teile dieser Nukleotidsequenz umfassen,
e) DNA-Sequenzen, die ein Derivat, Analog oder Fragment einer Nukleotidsequenz von a), b), c) oder d) darstellen.

Der Begriff "Hybridisierung" bedeutet im Rahmen dieser Erfindung eine Hybridisierung unter konventionellen Hybridisierungsbedingungen, vorzugsweise unter stringenten Bedingungen, wie sie beispielsweise in Sambrook *et al.* (1989. vide supra) beschrieben sind.

DNA-Sequenzen. die mit den DNA-Sequenzen, die ein Protein mit der enzymatischen Aktivität einer Saccharoseisomerase kodieren, hybridisieren, können z.B. aus genomischen oder cDNA-Bibliotheken eines beliebigen Organismus. der Saccharoseisomerase-DNA-Sequenzen natürlicherweise besitzt, isoliert werden. Die Identifizierung und Isolierung derartiger DNA-Sequenzen kann dabei z.B. unter Verwendung von DNA-Sequenzen erfolgen, die exakt oder im wesentlichen die in SEQ ID No. 1 angegebene Nukleotidsequenz oder Teile davon oder eine der oben erwähnten eine Saccharoseisomerase kodierenden Nukleotidsequenzen des Standes der Technik oder Teile dieser Sequenzen aufweisen, bzw. der reversen Komplemente dieser DNA-Sequenzen, z.B. mittels Hybridisierung nach Standardverfahren (siehe z.B. Sambrook *et al.* (1989), vide supra). Bei den als Hybridisierungssonde verwendeten Fragmenten kann es sich auch um synthetische Fragmente handeln, die mit Hilfe üblicher Synthesetechniken hergestellt wurden und deren Sequenz im wesentlichen mit einer der oben erwähnten Saccharoseisomerase-DNA-Sequenzen oder einem Teil davon übereinstimmt. Die DNA-Sequenzen, die ein Protein mit der enzymatischen Aktivität einer Saccharoseisomerase kodieren, umfassen auch DNA-Sequenzen, deren Nukleotidequenzen zu der einer der vorstehend beschriebenen DNA-Sequenzen degeneriert ist. Die Degeneration des genetischen Codes bietet dem Fachmann u.a. die Möglichkeit, die Nukleotidsequenz der DNA-Sequenz an die Codonpräferenz (codon usage) der Zielpflanze, also der aufgrund der Expression der Saccharoseisomerase-DNA-Sequenz nicht-kariogene Zucker produzierenden Pflanze bzw. Pflanzenzelle anzupassen und die Expression dadurch zu optimieren.

Die oben beschriebenen DNA-Sequenzen umfassen auch Fragmente, Derivate und allelische Varianten der oben beschriebenen DNA-Sequenzen, die ein Protein mit der enzymatischen Aktivität einer Saccharoseisomerase kodieren. Unter "Fragmenten" werden dabei Teile der DNA-Sequenz verstanden, die lang genug sind, um eines der beschriebenen Proteine zu kodieren. Der Ausdruck "Derivat" bedeutet in diesem Zusammenhang, dass die Sequenzen sich von den oben beschriebenen DNA-Sequenzen an einer oder mehreren Positionen unterscheiden, aber einen hohen Grad an Homologie zu diesen Sequenzen aufweisen. Homologie bedeutet dabei eine Sequenzidentität von mindestens 40 Prozent, insbesondere eine Identität von mindestens 60 Prozent, vorzugsweise über 80 Prozent und besonders bevorzugt über 90 Prozent. Dabei weisen die durch diese DNA-Sequenzen kodierten Proteine eine Sequenzidentität zu der in SEQ ID No. 2 und 3 angegebenen Aminosäuresequenz von mindestens 80 Prozent, vorzugsweise 85 Prozent und besonders bevorzugt über 90 Prozent, 95 Prozent und 98 Prozent auf. Die Abweichungen zu den oben beschriebenen DNA-Sequenzen können dabei beispielsweise durch Deletion, Substitution, Insertion oder Rekombination entstanden sein.

Bei den DNA-Sequenzen, die homolog zu den oben beschriebenen Sequenzen sind und Derivate dieser Sequenzen darstellen, handelt es sich in der Regel um Variationen dieser Sequenzen, die Modifikationen darstellen, die dieselbe biologische Funktion ausüben. Es kann sich dabei sowohl um natürlicherweise auftretende Variationen handeln, beispielsweise um Sequenzen aus anderen Organismen oder um Mutationen, wobei diese Mutationen auf natürliche Weise aufgetreten sein können oder durch gezielte Mutagenese eingeführt wurden. Ferner kann es sich bei den Variationen um synthetisch hergestellte Sequenzen handeln. Bei den allelischen Varianten kann es sich sowohl um natürlich auftretende Varianten handeln. als auch um synthetisch hergestellte oder durch rekombinante DNA-Techniken erzeugte Varianten.

In einer besonders bevorzugten Ausführungsform stammt die beschriebene, für eine Saccharoseisomerase kodierende DNA-Sequenz aus *Erwinia rhapontici.*

Für die Expression der in den erfindungsgemäßen rekombinanten Nukleinsäuremolekülen enthaltenen DNA-Sequenz in pflanzlichen Zellen ist diese mit regulatorischen Sequenzen verknüpft, die die Transkription in pflanzlichen Zellen gewährleisten. Hier kommt jeder in pflanzlichen Zellen aktive Promotor in Frage.

Dabei kann der Promotor so gewählt sein, dass die Expression konstitutiv erfolgt oder nur in einem bestimmten Gewebe oder Organ, zu einem bestimmten Zeitpunkt der Pflanzenentwicklung und/oder zu einem durch äußere Einflüsse, biotische oder abiotische Stimuli bestimmten Zeitpunkt (induzierte Genexpression). In Bezug auf die zu transformierende Pflanze kann der Promotor homolog oder heterolog sein.

Geeignete Promotoren sind z.B. der 35S RNA-Promotor des Cauliflower Mosaic Virus und der Ubiquitin-Promotor aus Mais für eine konstitutive Expression, ein Promotor, der eine Expression lediglich in photosynthetisch aktiven Geweben garantiert, z.B. der ST-LS1-Promotor (Stockhaus *et al.* (1987) Proc. Natl. Acad. Sci. USA 84:7943-7947; Stockhaus *et al.* (1989) EMBO J. 8:2445-2451 ) oder ein Promotor, der während der Pflanzentransformation. der Pflanzenregeneration oder bestimmten Stadien dieser Prozesse aktiv ist, wie z.B. zellteilungsspezifische Promotoren wie der Histon H3-Promotor (Kapros *et al.* (1993) InVitro Cell Cev. Biol. Plant 29:27-32) oder das chemisch induzierbare Tet-Repressor-System (Gatz *et al.* (1991) Mol. Gen. Genet. 227:229-237). Weitere geeignete Promotoren können der Literatur, z.B. Ward (1993, Plant Mol. Biol. 22:361-366), entnommen werden. Gleiches gilt für induzierbare und zell- bzw. gewebespezifische Promotoren, wie Meristem-spezifische Promotoren, die ebenfalls in der Literatur beschrieben worden sind und im Rahmen der Erfindung ebenfalls geeignet sind.

In einer bevorzugten Ausführungsform steht die Saccharoseisomerase-DNA-Sequenz unter Kontrolle eines organ- bzw. gewebespezifischen Promotors. Besonders bevorzugt handelt es sich hierbei um einen Promotor, der Expression in pflanzlichen Speicherorganen, wie z.B. Kartoffelknollen, liefert. Hier eignet sich z.B. der B33-Promotor des Klasse I Patatin-Gens aus *Solanum tuberosum* (Rocha-Sosa *et al.* (1989) EMBO J. 8:23-29). Der Promotor des Klasse I Patatin-Gens ist in Knollen ca. 100 bis 1000 mal aktiver als in Blättern (Rocha-Sosa *et al.,* vide supra). Weitere Gene mit knollenspezifischer oder zumindest in Knollen verstärkter Expression sind dem Fachmann aus der Literatur bekannt. Darin eingeschlossen sind z.B. ADP-Glukose-Pyrophosphorylase-Gene (kodierend für kleine und große Untereinheiten; Müller *et al.* (1990) Mol. Gen. Genet. 224:136-146). Andere bevorzugte Promotoren. umfassen solche, die vor allem mit der Expression von Stärke bildenden/modifizierenden Enzymen verbunden sind, oder die z.B. in Abhängigkeit von der Knollenentwicklung aktiv sind. Als Beispiele können hier genannt werden: Promotoren von Stärkesynthase-Genen und Genen, die für Verzweigungsenzyme kodieren (WO92/14827, WO92/11375 Der Durchschnittsfachmann kann darüber hinaus ohne weiteres zusätzliche Beispiele für Gene mit geeigneten Expressionsmustem der Literatur entnehmen.

Darüber hinaus ist der Durchschnittsfachmann in der Lage, mittels Routinemethoden weitere geeignete Promotoren zu isolieren. So kann der Fachmann mit Hilfe gängiger molekularbiologischer Methoden, z.B. Hybridisierungsexperimenten oder DNA-Protein-Bindungsstudien, Speicherorgan-spezifische regulatorische Nukleinsäurelemente identifizieren. Dabei wird z.B. in einem ersten Schritt aus Speicherorgangewebe des gewünschten Organismus, aus dem die regulatorischen Sequenzen isoliert werden sollen, die gesamte poly(A)⁺-RNA isoliert und eine cDNA-Bank angelegt. In einem zweiten Schritt werden mit Hilfe von cDNA-Klonen, die auf poly(A)⁺-RNA-Molekülen aus einem Nicht-Speicherorgangewebe basieren, aus der ersten Bank mittels Hybridisierung diejenigen Klone identifiziert, deren korrespondierende poly(A)⁺-RNA-Moleküle lediglich im Gewebe des Speicherorgans akkumulieren. Anschließend werden mit Hilfe dieser so identifizierten cDNAs Promotoren isoliert, die über Speicherorgan-spezifische regulatorische Elemente verfügen. Dem Fachmann stehen darüber hinaus weitere auf PCR basierende Methoden für die Isolierung geeigneter Speicherorgan-spezifischer Promotoren zur Verfügung.

In einer besonders bevorzugten Ausführungsform ist der Promotor der Promotor des Klasse I Patatin-Gens B33 aus Kartoffel.

Weiterhin bevorzugte Promotoren sind solche, die insbesondere in Früchten aktiv sind. Hierzu zählen bspw. der Promotor eines Polygalacturonase-Gens, z.B. aus Tomate, der während der Reife von Tomatenfrüchten Expression vermittelt (Nicholass *et al.* (1995) Plant Mol. Biol. 28:423-435; dieser Stand der Technik beschreibt die Analyse von Promotor/GUS-Fusionskonstrukten), der Promotor einer ACC-Oxidase, z.B. aus Apfel, der in transgenen Tomaten Reife- und Fruchtspezifität vermittelt (Atkinson *et al.* (1998) Plant Mol. Biol. 38:449-460; dieser Stand der Technik offenbart ebenfalls Promotor/GUS-Expressionsanalysen), oder der 2A11-Promotor aus Tomate (van Haaren *et al.* (1991) Plant Mol. Biol. 17:615-630, beschreibt ebenfalls Promotor/GUS-Fusionen). Aber auch der Patatin B33-Promotor, der überraschenderweise in Tomatenfrüchten aktiv ist (US-Patent 5,723,757).

Auch im Fall von Frucht-spezifischen Promotoren kann der Fachmann weitere geeignete Promotoren der Literatur entnehmen oder, wie oben für Speicherorgan-spezifische Promotoren beschrieben, mittels Routineverfahren isolieren.

In einer weiteren bevorzugten Ausführungsform ist die kodierende Region der Saccharoseisomerase operativ mit regulatorischen Elementen eines Promotors verbunden, der besonders in Samen aktiv ist, insbesondere während der Speicherphase. Hier sind als Beispiele zu nennen: der USP-Promotor (Bäumlein *et al.* 1991, Mol. Gen. Genet. 225:459-467; Phillips *et al.* (1997) EMBO J. 16:4489-4496), der Hordein-Promotor (Brandt *et al.* 1985, Carlsberg Res. Commun. 50:333-345), der Phaseolin-Promotor, z.B. aus Phaseolus vulgaris, der in sich entwickelnden Embryos aktiv ist und für den Promotor/GUS-Fusionsanalysen von van der Geest *et al.* (1996, Plant Mol. Biol. 32:579-588) beschrieben wurden, der Napin-Promotor, der ACP-Promotor und die FatB3- und FatB4-Promotoren, die dem auf dem Gebiet der pflanzlichen Molekularbiologie tätigen Fachmann wohl bekannt sind.

Auch im Fall von Samen-spezifischen Promotoren kann der Fachmann auf einfache Weise weitere Promotoren, die sich zur Expression der Saccharoseisomerase in Samen- und Speichergeweben eignen, vorzugsweise Promotoren von Genen, die Speicherproteine kodieren, der Literatur entnehmen, oder mittels Routineverfahren selbst aus beliebigen Organismen isolieren.

Ferner sind Transkriptions- bzw. Terminationssequenz vorhanden, die der korrekten Beendigung der Transkription dient, sowie der Addition eines polyA-Schwanzes an das Transkript dienen kann, dem eine Funktion bei der Stabilisierung der Transkripte beigemessen wird. Derartige Elemente sind in der Literatur beschrieben (z.B. Gielen (1989) EMBO J. 8:23-29) und beliebig austauschbar. z.B. der Terminator des Octopinsynthasegens aus *Agrobacterium tumefaciens.*

Die Erfindung betrifft weiterhin Vektoren, die erfindungsgemäße Nukleinsäuremoleküle enthalten und deren Verwendung die Produktion von nicht-kariogenen Zuckern in transformierten Pflanzenzellen und Pflanzen ermöglicht. Dabei handelt es sich insbesondere um Plasmide, Cosmide, Viren, Bakteriophagen und andere in der Gentechnik gängige Vektoren.

Zur Vorbereitung der Einführung fremder Gene in höhere Pflanzen bzw. deren Zellen stehen eine große Anzahl von Klonierungsvektoren zur Verfügung, die ein Replikationssignal für *E. coli* und ein Markergen zur Selektion transformierter Bakterienzellen enthalten. Beispiele für derartige Vektoren sind pBR322, pUC-Serien, M13mp-Serien, pACYC184 usw. Die gewünschte Sequenz kann an einer passenden Restriktionsschnittstelle in den Vektor eingeführt werden. Das erhaltene Plasmid wird dann für die Transformation von *E. coli*-Zellen verwendet. Transformierte *E. coli-*Zellen werden in einem geeigneten Medium gezüchtet und anschließend geerntet und lysiert, und das Plasmid wird wiedergewonnen. Als Analysenmethode zur Charakterisierung der gewonnenen Plasmid-DNA werden im allgemeinen Restriktionsanalysen, Gelelektrophoresen und weitere biochemischmolekularbiologische Methoden eingesetzt. Nach jeder Manipulation kann die Plasmid-DNA gespalten und gewonnene DNA-Fragmente mit anderen DNA-Sequenzen verknüpft werden.

Die erfindungsgemäßen Vektoren und Nukleinsäuremoleküle können weitere regulatorische Sequenzen und/oder Funktionseinheiten besitzen, die z.B. als Enhancer wirken oder eine Stabilisierung des Vektors in der Wirtszelle bewirken. Wie oben erwähnt, können die kodierenden Sequenzen auch durch Signalsequenzen ergänzt sein, die für den Transport des Genprodukts, also vorliegend des Proteins mit Saccharoseisomerase-Aktivität, zu einem bestimmten Kompartiment sorgen.

In einer bevorzugten Ausführungsform der Erfindung sorgen Signalsequenzen dafür, das die Saccharoseisomerase in die Zellwand bzw. den Apoplasten der transformierten Pflanzenzellen transportiert wird, d.h. die transformierten Pflanzen exprimieren eine chimäre Saccharoseisomerase, die ein Signalpeptid für den Transport in das Endoplasmatische Retikulum umfasst.

Geeignete Signalsequenzen, die die Aufnahme in das Endoplasmatische Retikulum gewährleisten, kann der Fachmann der einschlägigen Literatur entnehmen. Besonders geeignet ist bspw. die für das Signalpeptid des Proteinase-Inhibitor II-Gens aus Kartoffel kodierende Sequenz (Keil *et al.* (1996) Nucl. Acids Res. 14:5641-5650; Genbank Accession No. X04118).

Andere geeignete Signalsequenzen sorgen z.B. für die Aufnahme der Saccharoseisomerase in die Vakuole. Hier ist als Beispiel das Signalpeptid des Patatin-Gens aus Kartoffel (Rosahl *et al.* (1990) Plant Sci. 66:221-230) zu nennen.

Die Erfindung betrifft auch Wirtszellen, die die erfindungsgemäßen rekombinanten Nukleinsäuremoleküle oder Vektoren enthalten, wobei die Moleküle oder Vektoren transient oder stabil vorliegen können. Wirtszelle kann dabei jede Zelle und jeder Organismus sein, die bzw. der in der Lage ist, rekombinante DNA aufzunehmen und ggf. aufgenommene DNA-Sequenz zu exprimieren. Hier kommen prokaryontische und eukaryontische Zellen bzw. Organismen in Frage, insbesondere Mikroorganismen wie Bakterien, Viren, Pilze, Hefen und Algen, aber auch Pflanzenzellen, die die erfindungsgemäßen Nukleinsäuremoleküle oder Vektoren oder Teile oder Derivate davon enthalten. Vorzugsweise sind diese Pflanzenzellen aufgrund der Aufnahme der erfindungsgemäßen, eine Saccharoseisomerase kodierenden DNA-Sequenzen in der Lage, Proteine mit der enzymatischen Aktivität einer Saccharoseisomerase zu synthetisieren.

Durch die Bereitstellung der erfindungsgemäßen rekombinanten Nukleinsäuremoleküle und Vektoren ist es möglich, nicht-kariogene Zucker, insbesondere Palatinose und Trehalulose, in Pflanzenzellen zu produzieren. Die vorliegende Erfindung betrifft somit auch ein Verfahren zur Produktion von nicht-kariogenen Zuckern in transformierten Pflanzenzellen, umfassend die Schritte:
i) Herstellung eines rekombinanten Nukleinsäuremoleküls, umfassend
   a) regulatorische Sequenzen eines in Pflanzenzellen aktiven Promotors;
   b) operativ daran gebunden eine DNA-Sequenz, die ein Protein mit der enzymatischen Aktivität einer Saccharoseisomerase kodiert; und
   c) operativ daran gebunden regulatorische Sequenzen, die als Transkriptions-, Terminations- und/oder Polyadenylierungssignale in Pflanzenzellen dienen können,
   oder eines Vektors, enthaltend ein solches rekombinantes Nukleinsäuremolekül; und
ii) Übertragung des Nukleinsäuremoleküls aus i) auf Pflanzenzellen.

Optional können im Anschluß an die Übertragung des Nukleinsäuremoleküls auf Pflanzenzellen aus den transformierten Pflanzenzellen transgene Pflanzen regeneriert werden. Die Regeneration ganzer Pflanzen und ihre Verwendung als Produktionsstätte für nicht-kariogene Zucker ist aber nicht immer erforderlich. So können auch Pflanzenzellen, Pflanzenkalli, Pflanzenteile und -organe als Produktionsstätte genutzt werden, bspw. in Form von pflanzlichen Suspensionskulturen.

Voraussetzung für die Einführung der erfindungsgemäßen rekombinanten Nukleinsäuremoleküle und Vektoren in Pflanzenzellen ist die Verfügbarkeit geeigneter Transformationssysteme. Hier wurde während der letzten zwei Jahrzehnte ein breites Spektrum an Transformationsmethoden entwickelt und etabliert. Diese Techniken umfassen die Transformation pflanzlicher Zellen mit T-DNA unter Verwendung von *Agrobacterium tumefaciens* oder *Agrobacterium rhizogenes* als Transformationsmittel, Diffusion von Protoplasten, den direkten Gentransfer isolierter DNA in Protoplasten, die Injektion und Elektroporation von DNA in Pflanzenzellen, die Einbringung von DNA mittels der biolistischen Methoden sowie weitere Möglichkeiten, wobei der Fachmann die jeweils geeignete Methode problemlos ermitteln kann. Sämtliche Transformationsverfahren sind seit vielen Jahren gut etabliert und gehören zweifelsohne zum Standardrepertoire des Fachmanns in der pflanzlichen Molekularbiologie, Pflanzenbiotechnologie und Zellund Gewebekultur.

Bei der Injektion und Elektroporation von DNA in Pflanzenzellen werden per se keine speziellen Anforderungen an die verwendeten Plasmide gestellt. Ähnliches gilt für den direkten Gentransfer. Es können einfache Plasmide, wie z.B. pUC-Derivate, verwendet werden. Sollen aber aus derartig transformierten Zellen ganze Pflanzen regeneriert werden, ist die Anwesenheit eines selektierbaren Markergens empfehlenswert. Dem Fachmann sind die gängigen Selektionsmarker bekannt, und es stellt für ihn kein Problem dar, einen geeigneten Marker auszuwählen.

Je nach Einführungsmethode gewünschter Gene in die Pflanzenzelle können weitere DNA-Sequenzen erforderlich sein. Werden z.B. für die Transformation der Pflanzenzelle das Ti- oder Ri-Plasmid verwendet, so muss mindestens die rechte Begrenzung. häufig jedoch die rechte und linke Begrenzung der im Ti- bzw. Ri-Plasmid enthaltenen T-DNA als Flankenbereich mit den einzuführenden Genen verbunden werden. Werden für die Transformation Agrobakterien verwendet, muss die einzuführende DNA in spezielle Plasmide kloniert werden, und zwar entweder in einen intermediären oder in einen binären Vektor. Die intermediären Vektoren können aufgrund von Sequenzen, die homolog zu Sequenzen in der T-DNA sind, durch homologe Rekombination in das Ti- oder Ri-Plasmid der Agrobakterien integriert werden. Dieses enthält außerdem die für den Transfer der T-DNA notwendige vir-Region. Intermediäre Vektoren können nicht in Agrobakterien replizieren. Mittels eines Helferplasmids kann der intermediäre Vektor auf *Agrobacterium tumefaciens* übertragen werden (Konjugation). Binäre Vektoren können sowohl in *E. coli* als auch in Agrobakterien replizieren. Sie enthalten ein Selektionsmarkergen und einen Linker oder Polylinker, welche von der rechten und linken T-DNA-Grenzregion eingerahmt werden. Sie können direkt in die Agrobakterien transformiert werden. Das als Wirtszelle dienende Agrobakterium soll ein Plasmid, das eine vir-Region trägt, enthalten. Die vir-Region ist für den Transfer der T-DNA in die Pflanzenzelle notwendig. Zusätzliche T-DNA kann vorhanden sein. Das derartig transformierte Agrobakterium wird zur Transformation von Pflanzenzellen verwendet. Die Verwendung von T-DNA für die Transformation von Pflanzenzellen ist intensiv untersucht und ausreichend in allseits bekannten Übersichtsartikeln und Handbüchern zur Pflanzentransformation beschrieben worden.

Für den Transfer der DNA in die Pflanzenzelle können Pflanzen-Explantate zweckmäßigerweise mit *Agrobacterium tumefaciens* oder *Agrobacterium rhizogenes* kultiviert werden. Aus dem infizierten Pflanzenmaterial (z.B. Blattstücke. Stengelsegmente, Wurzeln, aber auch Protoplasten oder Suspensions-kultivierte Pflanzenzellen) können dann in einem geeigneten Medium, welches Antibiotika oder Biozide zur Selektion transformierter Zellen enthalten kann, wieder ganze Pflanzen regeneriert werden.

Ist die eingeführte DNA einmal im Genom der Pflanzenzelle integriert, so ist sie dort in der Regel stabil und bleibt auch in den Nachkommen der ursprünglich transformierten Zelle erhalten. Sie enthält normalerweise einen Selektionsmarker, der den transformierten Pflanzenzellen Resistenz gegenüber einem Biozid oder einem Antibiotikum wie Kanamycin, G 418, Bleomycin, Hygromycin, Methotrexat, Glyphosat, Streptomycin. Sulfonylharnstoff, Gentamycin oder Phosphinotricin u.a. vermittelt. Der individuell gewählte Marker sollte daher die Selektion transformierter Zellen gegenüber Zellen. denen die eingeführte DNA fehlt, gestatten. Hierzu sind auch alternative Marker geeignet. wie nutritive Marker. Screeningmarker (wie GFP, green fluorescent protein). Selbstverständlich kann auch vollkommen auf Selektionsmarker verzichtet werden, was allerdings mit einem ziemlich hohen Screeningbedarf einhergeht. Falls der eingesetzte Selektionsmarker nach erfolgter Transformation und Identifizierung erfolgreich transformierter Zellen bzw. Pflanzen wieder entfernt werden soll, stehen dem Fachmann hierfür verschiedene Strategien zur Verfügung. So können z.B. sequenzspezifische Rekombinasen verwendet werden, z.B. in Form der Retransformation einer Rekombinase-exprimierenden Ausgangslinie und Auskreuzung der Rekombinase nach erfolgter Entfernung des Selektionsmarkers (siehe z.B. Reiss *et al.* (1996) Proc. Natl. Acad. Sci. USA 93:3094-3098; Bayley *et al.* (1992) Plant Mol. Biol. 18:353-361; Lloyd *et al.* (1994) Mol. Gen. Genet. 242:653-657; Mäser *et al.* (1991) Mol. Gen. Genet. 230:170-176; Onouchi *et al.* (1991) Nucl. Acids Res. 19:6373-6378). Der Selektionsmarker kann auch durch Cotransformation mit anschließender Auskreuzung entfernt werden.

Die Regeneration der transgenen Pflanzen aus transgenen Pflanzenzellen erfolgt nach üblichen Regenerationsmethoden unter Verwendung üblicher Nährmedien und Phytohormone. Die so erhaltenen Pflanzen können dann, falls erwünscht, mittels üblicher Verfahren, einschließlich molekularbiologischer Methoden, wie PCR, Blot-Analysen, oder biochemischer Verfahren auf Anwesenheit der eingeführten DNA, die ein Protein mit der enzymatischen Aktivität einer Saccharoseisomerase kodiert, bzw. auf Anwesenheit von Saccharoseisomerase-Enzymaktivität untersucht werden.

Des weiteren betrifft die Erfindung Pflanzenzellen, die ein erfindungsgemäßes Nukleinsäuremolekül oder einen erfindungsgemäßen Vektor mit Saccharoseisomerase-DNA-Sequenzen enthalten und nicht-kariogene Zucker, insbesondere Palatinose und/oder Trehalulose synthetisieren.

Gegenstand der Erfindung sind auch die durch Regeneration der erfindungsgemäßen Pflanzenzellen erhältlichen transgenen Pflanzen. Bei der transgenen Pflanze bzw. den transgenen Pflanzenzellen kann es sich um jede beliebige monokotyle oder dikotyle Pflanze bzw. Pflanzenzelle handeln, vorzugsweise handelt es sich um Nutzpflanzen bzw. Zellen von Nutzpflanzen. Besonders bevorzugt handelt es sich um Pflanzen mit Speicherorganen, wie z.B. Kartoffel. Ebenfalls bevorzugt sind Zuckerrübe, Tomate, Banane, Karotte, Zuckerrohr, Mais, Erdbeere, Ananas, Papaya. Am meisten bevorzugt sind Zuckerrübe und Zuckerrohr.

Die Erfindung betrifft ebenfalls Ernteprodukte und Vermehrungsmaterial transgener Pflanzen, deren Zellen oder Geweben ein erfindungsgemäßes Nukleinsäuremolekül enthalten und nicht-kariogene Zucker synthetisieren. Bei den Emteprodukten und dem Vermehrungsmaterial handelt es sich insbesondere um Früchte, Samen, Knollen, Wurzelstöcke, Sämlinge, Stecklinge, etc.

Unabhängig von den verwendeten regulatorischen Sequenzen, unter deren Kontrolle die Expression der Saccharoseisomerase-DNA-Sequenzen steht, steht dem Fachmann ein breites Spektrum an molekularbiologischen und/oder biochemischen Verfahren für die Analyse der transformierten Pflanzenzellen. transgenen Pflanzen, Pflanzenteile, Ernteprodukte und Vermehrungsmaterial zur Verfügung, z.B. PCR, Northem Blot-Analyse zum Nachweis Saccharoseiosmerase-spezifischer RNA bzw. zur Bestimmung der Höhe der Akkumulation von Saccharoseisomerase-spezifischer RNA. Southem Blot-Analyse zur Identifizierung von Saccharoseisomerasekodierenden DNA-Sequenzen oder Westem Blot-Analyse zum Nachweis des durch die erfindungsgemäßen Nukleinsäuremoleküle kodierten Saccharoseisomerase. Selbstverständlich kann der Nachweis der enzymatischen Aktivität der Saccharoseisomerase auch vom Fachmann mittels in der Literatur erhältlicher Protokolle bestimmt werden. Auch die Produkte der von der Saccharoseisomerase katalysierten Umlagerung, nämlich insbesondere Palatinose und/oder Trehalulose kann der Fachmann nachweisen. Weiter kann man z.B. den durch Selbstung oder Kreuzungen erhaltenen Samen auf Medium auslegen, das das zu dem zusammen mit der Saccharoseisomerase-DNA-Sequenz übertragenen Selektionsmarker passende Selektionsmittel enthält, und anhand der Keimfähigkeit und des Wachstums der Tochtergeneration(en) und des Segregationsmusters Rückschlüsse auf den Genotyp der jeweiligen Pflanze schließen.

Gegenstand der Erfindung ist auch ein Verfahren zur Herstellung von Pflanzenzellen. die nicht-kariogene Zucker, insbesondere Palatinose und/oder Trehalulose produzieren, umfassend die Übertragung eines erfindungsgemäßen Nukleinsäuremoleküls oder eines erfindungsgemäßen Vektors auf Pflanzenzellen.

Weiter betrifft die Erfindung die Verwendung von DNA-Sequenzen, die ein Protein mit der enzymatischen Aktivität einer Saccharoseisomerase kodieren, für die Produktion nicht-kariogener Zucker in Pflanzenzellen und Pflanzen und die Herstellung von nicht-kariogenen Zucker produzierenden Pflanzenzellen und Pflanzen.

Die Erfindung basiert auf der Beobachtung, dass sich DNA-Sequenzen, die ein Protein mit der enzymatischen Aktivität einer Saccharoseisomerase kodieren, hervorragend für die Produktion nicht-kariogener Süßstoffe in transgenen Pflanzenzellen und Pflanzen nutzen lassen, was in den nachfolgenden Beispielen, die nur der Veranschaulichung der Erfindung dienen und in keiner Weise als Einschränkung zu verstehen sind, erläutert wird.

### Beispiele

### Gentechnische Verfahren, die den Ausführungsbeispielen zugrunde liegen:

### 1. Allgemeine Klonierungsverfahren

Klonierungsverfahren wie z.B.: Restriktionsspaltungen, DNA-Isolierung, Agarose-Gelelektrophorese, Reinigung von DNA-Fragmenten, Transfer von Nukleinsäuren auf Nitrozellulose und Nylon Membranen, Verknüpfen von DNA-Fragmenten, Transformation von *E. coli* Zellen, Anzucht von Bakterien, Sequenzanalyse rekombinanter DNA wurden nach Sambrook *et al.* (1989, vide supra) durchgeführt. Die Transformation von *Agrobacterium tumefaciens* wurde entsprechend der Methode von Höfgen und Willmitzer (1988, Nucl. Acids Res. 16:9877) ausgeführt. Die Anzucht der Agrobacterien erfolgte in YEB Medium (Vervliet *et al.* (1975) Gen. Virol. 26:33).

### 2. Erzeugung einer genomischen Bank von Erwinia rhapontici

Zur Herstellung einer genomischen Bank aus *Erwinia rhapontici* (DSM 4484) wurde chromosomale DNA aus den Zellen einer 50 ml-Übernachtkultur nach Standardprotokoll isoliert. Anschließend wurden ca. 300 µg der DNA mit dem Restriktionsenzym Sau3A partialverdaut und auf einem präparativen Agarosegel aufgetrennt. Fragmente zwischen 5 und 12 kb wurden mittels Qiaquick Gel Extraction Kit (Qiagen, Hilden) aus dem Gel eluiert. Die so erhaltenen DNA-Fragmente wurden in BamHI-verdaute Lambda ZAP-Express-Arme (Stratagene, La Jolla, USA) ligiert und anschließend *in vitro* verpackt (Gigapack III Gold Packaging Extract, Stratagene, nach Herstellerangaben). *E. coli*-Bakterien des Stammes XL-MRF' (Stratagene) wurden mit rekombinanten Lambda-Phagen infiziert, der Titer der Bank bestimmt und schließlich die Bank amplifiziert.

### 3. Durchmusterung einer genomischen Bank

Zur Isolierung von genomischen Klonen wurden ca. 10⁵ Phagen plattiert. Nach Transfer der Phagen auf Nylonfilter (Genescreen, NEN) wurden die Filter mit einem radioaktiv markierten DNA-Fragment hybridisiert. Positive Signale wurden mittels Autoradiographie sichtbar gemacht und vereinzelt.

### 4. Bakterienstämme und Plasmide

*E. coli* (XL-1 Blue, XL-MRF' und XLOLR)-Bakterien wurden von der Firma Stratagene bezogen. *Erwinia rhapontici* (DSM 4484) wurde von der Deutschen Sammlung für Mikroorganismen und Zellkulturen GmbH (Braunschweig, Deutschland) bezogen. Der zur Pflanzentransformation eingesetzte Agrobacterium-Stamm (C58C1 mit dem Plasmid pGV 3850kan) wurde von Debleare *et al.* (1985, Nucl. Acids Res. 13:4777) beschrieben.

Zur Klonierung wurden die Vektoren pCR-Blunt (Invitrogen, Niederlande), pMALc2 (New England Biolabs), pUC 19 (Yanish-Perron (1985) Gene 33;103-119) und Bin19 (Bevan (1984) Nucl. Acids Res. 12:8711-8720) verwendet.

### 5. Tabaktransformation

Zur Transformation von Tabakpflanzen *(Nicotiana tabacum* L. cv. Samsun NN) wurden 10ml einer unter Selektion gewachsenen Übernachtkultur von *Agrobacterium tumefaciens* abzentrifugiert. der Überstand verworfen, und die Bakterien im gleichen Volumen Antibiotika-freien Mediums resuspendiert. In einer sterilen Petrischale wurden Blattscheiben steriler Pflanzen (Durchmesser ca. 1 cm) in dieser Bakterienlösung gebadet. Anschließend wurden die Blattscheiben in Petrischalen auf MS-Medium (Murashige und Skoog (1962) Physiol. Plant 15:473) mit 2% Saccharose und 0,8% Bacto-Agar ausgelegt. Nach 2-tägiger Inkubation im Dunkeln bei 25°C wurden sie auf MS-Medium mit 100 mg/l Kanamycin, 500 mg/l Claforan, 1mg/l Benzylaminopurin (BAP), 0,2mg/l Naphthylessigsäure (NAA), 1,6% Glukose und 0,8% Bacto-Agar übertragen und die Kultivierung (16 Stunden Licht /8 Stunden Dunkelheit) fortgesetzt. Wachsende Sprosse wurden auf hormonfreies MS-Medium mit 2% Saccharose, 250 mg/l Claforan und 0,8% Bacto-Agar überführt.

### 6. Nachweis von Palatinose in Pflanzenextrakten

Zum Nachweis von Saccharoseisomerase-Aktivität in Pflanzenextrakten wurden Blattscheiben mit einem Durchmesser von ca. 0,8 cm für 2 h bei 70°C mit 100 µl 80 % Ethanol und 10 mM HEPES-Puffer (pH 7,5) extrahiert. Für die Analyse eines Aliquots dieser Extrakte wurde ein HPLC-System der Firma Dionex verwendet, welches mit einer PA-1 (4 x 250 mm)-Säule und einem gepulsten elektrochemischen Detektor ausgestattet war. Vor der Injektion wurden die Proben für 2 Minuten bei 13.000 rpm abzentrifugiert. Zucker wurden anschließend mit einem 10minütigen Gradienten von 0 bis 1 M Natriumacetat nach 4 Minuten bei 150 mM NaOH und einer Durchflussrate von 1ml/min eluiert. Zur Identifizierung und Quantifizierung der Zucker wurden die entsprechenden Standards der Firma Sigma verwendet.

### 7. Kartoffeltransformation

20 kleine, mit einem Skalpell verwundete Blätter einer Kartoffel-Sterilkultur wurden in 10 ml MS-Medium mit 2% Saccharose gelegt, welches 50 µl einer unter Selektion gewachsenen *Agrobacterium tumefaciens*-Übemachtkultur enthielt. Nach 5-minütigem, leichtem Schütteln wurden die Petrischalen bei 25 °C im Dunkeln inkubiert. Nach zwei Tagen wurden die Blätter auf MS-Medium mit 1.6% Glucose, 2 mg/l Zeatinribose, 0,02 mg/l Giberellinsäure, 500 mg/l Claforan, 50 mg/l Kanamycin und 0,8 % Bacto-Agar ausgelegt. Nach einwöchiger Inkubation bei 25 °C und 3000 Lux wurde die Claforankonzentration im Medium halbiert. Eine weitere Woche Kultivierung erfolgte unter bekannten Bedingungen (Rocha-Sosa et al. (1989) EMBO J. 8:23-29).

### Beispiel 1

### PCR-Amplifikation eines Subfragments der Saccharoseisomerase aus Erwinia rhaponitici

Die Klonierung eines Subfragments der Saccharoseisomerase erfolgte mittels Polymerase-Kettenreaktion (**P**olymerase **C**hain **R**eaction, PCR). Als Matrizenmaterial diente genomische DNA aus *E. rhapontici* (DSM 4484), die nach Standardprotokoll isoliert wurde. Die Amplifizierung erfolgte unter Verwendung der spezifischen Primer
- FB 83 5'-GGATCCGGTACCGTTCAGCAATCAAAT-3' und
- FB 84 5'-GTCGACGTCTTGCCAAAAACCTT-3'.
die von einer Saccharoseisomerase-Sequenz des Standes der Technik abgeleitet wurden. Primer FB 83 umfaßt die Basen 109 - 127 und Primer FB 84 die Basen 1289 - 1306 der kodierenden Region des Saccharoseisomerase-Gens von *E. rhapontici.* Das PCR-Reaktionsgemisch (100 µl) enthielt chromosomale Bakterien DNA (1µg), Primer FB 83 und FB 84 (jeweils 250 ng), Pfu DNA-Polymerase-Reaktionspuffer (10 µl, Stratagene), 200 µM dNTPs (dATP, dCTP, dGTP, dTTP) und 2,5 Einheiten Pfu DNA-Polymerase (Stratagene). Vor Beginn der Amplifikationszyklen wurde das Gemisch für 5 min auf 95 °C erhitzt. Die Polymerisierungsschritte (30 Zyklen) wurden in einem automatischen T3-Thermocycler (Biometra) nach folgendem Programm durchgeführt: Denaturierung 95 °C (1 Minute), Anlagerung der Primer bei 55 °C (40 Sekunden), Polymerase-Reaktion bei 72 °C (2 Minuten). Das erhaltene Fragment wurde in den Vektor pCR-Blunt (Invitrogen) kloniert. Die Identität der amplifizierten DNA wurde mittels Sequenzanalyse verifiziert.

Das amplifizierte Subfragment kann gut als Hybridisierungssonde für die Isolierung weiterer Saccharoseisomerase-DNA-Sequenzen aus anderen Organismen oder als Sonde bei der Analyse transgener Zellen und Pflanzen eingesetzt werden.

### Beispiel 2

### PCR-Amplifikation einer Saccharoseisomerase aus Erwinia rhaponitici

Unter Verwendung des in Beispiel 1 amplifizierten Fragmentes wurde eine genomisch Bank nach Standardmethoden durchmustert. Anschließende Sequenzanalysen erlaubten die Bestimmung des offenen Leserahmens der Saccharoseisomerase; Von dieser Sequenz wurden die Oligonukleotidprimer-FB 83, FB 96 und FB 97 abgeleitet.

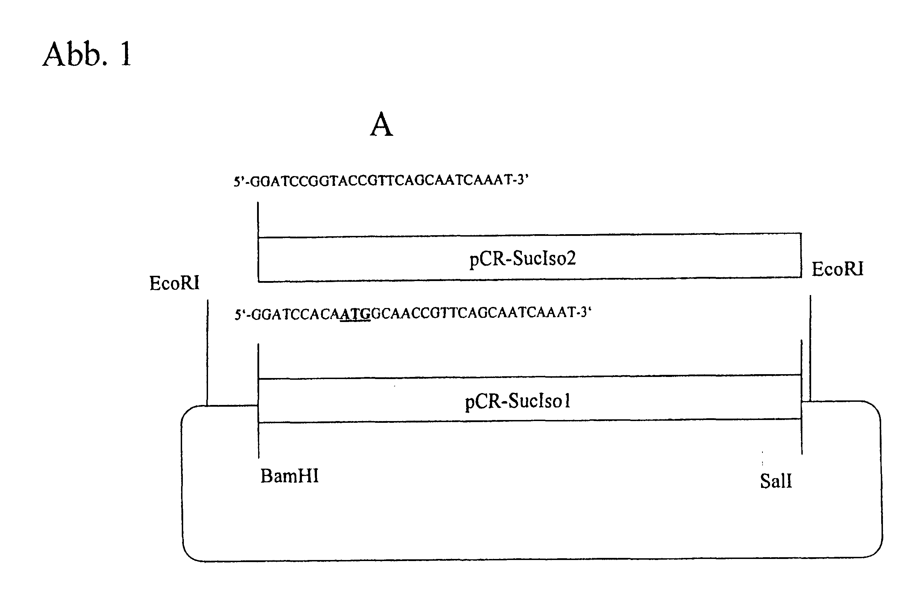

Die Klonierung des vollständingen offenen Leserasters der Saccharose-Isomerase erfolgte mittels Polymerase-Kettenreaktion (**P**olymerase **C**hain **R**eaction, PCR). Als Matrizenmaterial diente genomische DNA aus *E. rhapontici* (DSM 4484), die nach Standardprotokoll isoliert wurde. Die Amplifizierung erfolgte unter Verwendung der spezifischen Primer und für pCR-SucIso1. Primer FB 96 umfaßt die Basen 109 - 127 und beinhaltet zusätzlich ein Startkodon, Primer FB 97 die Basen 1786 - 1803 der kodierenden Region des Saccharoseisomerase-Gens. Zur Herstellung des Konstruktes pCR-SucIso2 wurde als 5'-Primer FB 83 (5'-GGATCCGGTACCGTTCAGCAATCAAAT-3'), welcher kein zusätzliches ATG enthält, verwendet. Zur Klonierung der amplifizierten DNA in Expressionsvektoren tragen die Primer zusätzlich folgende Restriktonsschnittstellen: Primer FB 96 bzw. FB 83, BamHI; Primer FB 97, SaII. Das PCR-Reaktionsgemisch (100 µl) enthielt chromosomale Bakterien-DNA (1 µg), Primer FB 96 und FB 97 für pCR-SucIso1, bzw. Primer FB 83 und FB 97 für pCR-SucIso2 (jeweils 250 ng), Pfu DNA-Polymerase-Reaktionspuffer (10 µl. Stratagene), 200 µM dNTPs (dATP, dCTP, dGTP, dTTP) und 2.5 Einheiten Pfu DNA-Polymerase (Stratagene). Vor Beginn der Amplifikationszyklen wurde das Gemisch für 5 min auf 95 °C erhitzt. Die Polymerisierungsschritte (30 Zyklen) wurden in einem automatischen T3-Thermocycler (Biometra) nach folgendem Programm durchgeführt: Denaturierung 95 °C (1 Minute), Anlagerung der Primer bei 55°C (40 Sekunden), Polymerase-Reaktion bei 72 °C (2 Minuten). Das amplifizierte Saccharoseisomerase-Fragment wurde in den Vektor pCR-Blunt (Invitrogen) kloniert, wodurch das Plasmid pCR-SucIso (mit Translationsstart) bzw. pCR-SucIso2 (ohne Translationsstart) erhalten wurde (siehe Abbildung 1). Die Identität der amplifizierten DNA wurde mittels Sequenzanalyse verifiziert.
Das Fragment A beinhaltet die Sequenz einer Saccharoseisomerase aus *E. rhapontici,* die sich von Nukleotid 109 - 1803 des Saccharoseisomerase-Gens erstreckt. Die Nukleotidsequenz der verwendeten Primer wurde jeweils hervorgehoben.

### Beispiel 3

### Herstellung von Plasmid p35S-cytIso

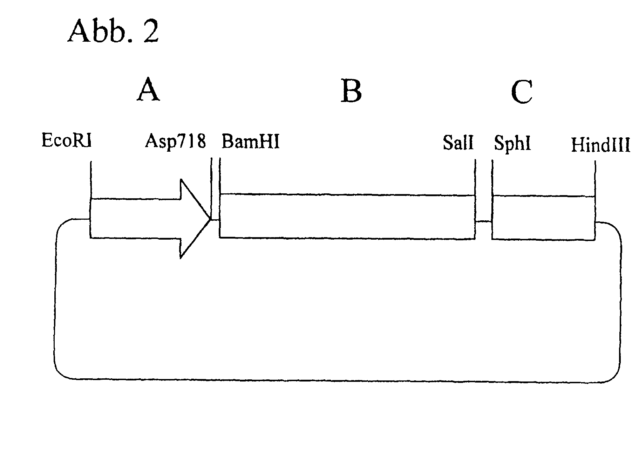

Eine DNA Sequenz, die für eine Saccharose-Isomerase kodiert, wurde aus dem Plasmid pCR-SucIso2 isoliert und mit dem 35S-Promotor des Cauliflower Mosaik Virus, der eine konstitutive Expression in transgenen Pflanzenzellen vermittelt, sowie einem pflanzlichen Terminationssignal versehen. Das pflanzliche Terminationssignal beinhaltet das 3'-Ende der Polyadenylierungsstelle des Octopin-Synthase Gens. Das Plasmid p35S-cytIso beinhaltet drei Fragmente A, B und C, die in die Schnittstellen für Restriktionsenzyme des Polylinkers von pUC 18 kloniert wurden (s. Abbildung 2).
Das Fragment A beinhaltet den 35S Promotor des Cauliflower Mosaik Virus (CaMV). Es enthält ein Fragment, welches die Nukleotide 6909 bis 7437 des CaMV umfaßt (Franck (1980) Cell 21:285) und wurde als EcoRI/KpnI-Fragment aus dem Plasmid pDH51 (Pietrzak *et al.* (1986) Nucl. Acids Res. 14:5857) isoliert und zwischen die EcoRI- und KpnI-Schnittstelle des Plasmids pUC18 kloniert.
Das Fragment B enthält die kodierende Region der Saccharoseisomerase von Basenpaar 109 - 1803, die als BamHI/SalI Fragment aus dem Plasmid pCR-SucIso1 (s. Abb. 1) isoliert und zwischen die BamHI- und SaII-Schnittstelle des Polylinkers von pUC18 kloniert wurde.
Das Fragment C enthält das Polyadenylierungssignal des Gens 3 der T-DNA des Ti-Plasmids pTiACH5 (Gielen *et al.* (1984) EMBO J. 3:835), Nukleotide 11749 - 11939, welches als PvuII/HindIII-Fragment aus dem Plasmid pAGV 40 (Herrera-Estrella *et al.* (1983) Nature 303:209) isoliert und nach Addition von SphI-Linkern an die PvuII-Schnittstelle zwischen die SphI- und HindIII-Schnittstelle des Polylinkers von pUC 18 kloniert worden ist.
Das chimäre Gen wurde anschließend als EcoRI/HindIII-Fragment zwischen die EcoRI- und HindIII-Schnittstelle des Plasmids pBIN19 (Bevan (1984) Nucl. Acids Res. 12:8711) kloniert.

In p35S-cytlso (35S = 35S-Promotor, cyt = Cytosol, Iso = Saccharoseisomerase) steht die kodierende Region der Saccharoseisomerase aus *E. rhapontici* unter konstitutiver Kontrolle, das Genprodukt ist im Cytosol lokalisiert.

### Beispiel 4

### Herstellung von Plasmid p35S-cwIso

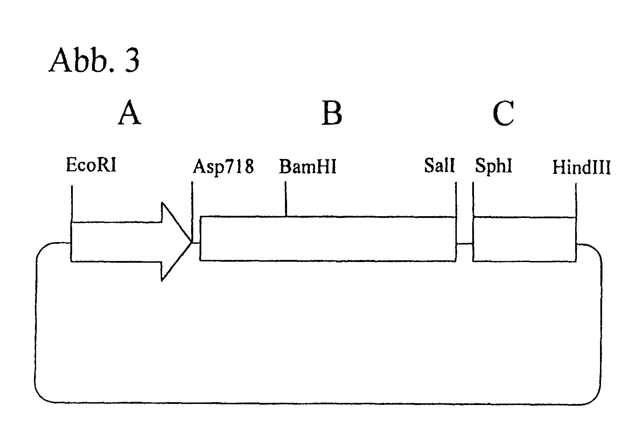

In analoger Verfahrensweise wie unter Beispiel 3 beschrieben, jedoch mit der Modifikation, dass vor die kodierende Sequenz des Saccharoseisomerase-Gens ein für die Aufnahme in das Endoplasmatische Retikulum notwendiges Signalpeptid eines pflanzlichen Gens (Proteinase-Inhibitor II-Gen aus Kartoffel (Keil *et al.* (1986) Nucl. Acids Res. 14:5641-5650; Genbank Accession X04118) fusioniert wurde, wurde das Plasmid p35S-cwIso hergestellt. Dazu wurde das Saccharoseisomerase-Fragment aus dem Konstrukt pCR-SucIso2 (siehe Abb. 1) über die Restriktionsschnittstellen BamHI und SaII herausgeschnitten und in einen BamHI/SaIIgeöffneten pMA-Vektor ligiert. Der Vektor pMA stellt eine modifizierte Form des Vektors pBinAR (Höfgen und Willmitzer (1990) Plant Sci. 66:221-230) dar, welche den 35 5-Promotor des Cauliflower Mosaik Virus, der eine konstitutive Expression in transgenen Pflanzen vermittelt, ein Signalpeptid des Proteinase-Inhibitors II aus Kartoffel, welches die Zielsteuerung des Fusionsproteins in die Zellwand vermittelt, sowie ein pflanzliches Terminationssignal enthält. Das pflanzliche Terminationssignal beinhaltet das 3'-Ende der Polyadenylierungsstelle des Octopin-Synthase Gens. Zwischen der Teilsequenz des Proteinase-Inhibitors und dem Terminationssignal befinden sich Schnittstellen für die Restriktionsenzyme BamHI, Xbal, SaII, PstI und Sphl (in dieser Reihenfolge), welche die Insertion entsprechender DNA-Fragmente ermöglichen, so dass ein Fusionsprotein zwischen dem Proteinase-Inhibitor und dem eingefügten Protein entsteht, welches dann in die Zellwand von transgenen Pflanzenzellen befördert wird, die dieses Protein expremieren (Abb.3). Das Fragment A beinhaltet den 355-Promotor des Cauliflower Mosaik Virus (CaMV). Es enthält ein Fragment. welches die Nukleotide 6909 bis 7437 des CaMV umfaßt (Franck (1980) Cell 21:285.
Das Fragment B enthält die Nukleotide 923 - 1059 eines Proteinase-Inhibitor II-Gens aus der Kartoffel (Keil *et al.*, supra), welcher über einen Linker mit der Sequenz ACC GAA TTG GG an das Saccharoseisomerase Gen aus *Erwinia rhapontici,* welches die Nukleotide 109 - 1803 umfaßt, fusioniert ist. Dadurch wird ein für die Aufnahme von Proteinen in das Endoplasmatische Retikulum (ER) notwendige Signalpeptid eines pflanzlichen Proteins N-terminal an die Saccharoseisomerase Sequenz fusioniert.
Das Fragment C enthält das Polyadenylierungssignal des Gens 3 der T-DNA des Ti-Plasmids pTiACH5 (Gielen *et al.* (1984) EMBO J. 3:835), Nukleotide 11749 - 11939.

In p35S-cwIso (35S = 35S-Promotor, cw = Zellwand, Iso = Saccharoseisomerase) steht die kodierende Region der Saccharoseisomerase aus *E. rhapontici* unter konstitutiver Kontrolle, das Genprodukt wird in das ER aufgenommen.

### Beispiel 5

### Herstellung von Plasmid p35S-vacIso

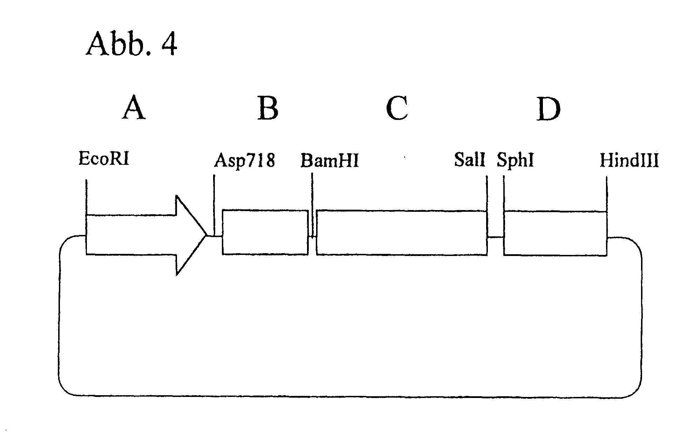

In analoger Verfahrensweise wie unter Beispiel 4 beschrieben, jedoch mit der Modifikation, dass vor die kodierende Sequenz des Saccharoseisomerase-Gens ein für die Aufnahme in die Vakuole notwendiges Signalpeptid eines pflanzlichen Gens (Patatin aus Kartoffel (Rosahl *et al* (1986) Mol. Gen. Genet. 203:214-220) fusioniert wurde, wurde das Plasmid p35S-vacIso hergestellt. Dazu wurde das Saccharoseisomerase-Fragment aus dem Konstrukt pCR-SucIso2 (siehe Abb. 1) über die Restriktionsschnittstellen BamHI und SaII herausgeschnitten und in eine modifizierte Version des Vektors pBinAR (Höfgen und Willmitzer, vide supra) ligiert, welche den 35S-Promotor des Cauliflower Mosaik Virus, der eine konstitutive Expression in transgenen Pflanzen vermittelt, ein Fragment des Patatin-Gens aus Kartoffel, welches die Nukleotide 34 - 1230 des transkribierten Bereiches des Patatin-Gens enthält (Rosahl *et al,* vide supra), was die Zielsteuerung des Fusionsproteins in die Vakuole vermittelt, sowie ein pflanzliches Terminationssignal . enthält. Das pflanzliche Terminationssignal beinhaltet das 3'-Ende der Polyadenylierungsstelle des Octopin-Synthase Gens. Zwischen der Teilsequenz des Patatin-Gens und dem Terminationsignal befinden sich Schnittstellen für die Restriktionsenzyme BamHI, XbaI, SaII, PstI und SphI (in dieser Reihenfolge), welche die Insertion entsprechender DNA-Fragmente ermöglichen. Hierdurch entsteht ein Fusionsprotein aus Patatin und der Saccharoseisomerase aus *E. rhapontici* (siehe Abbildung 4).
Das Fragment A beinhaltet den 35S-Promotor des Cauliflower Mosaik Virus (CaMV). Es enthält ein Fragment, welches die Nukleotide 6909 bis 7437 des CaMV umfaßt (Franck (1980) Cell 21:285).

Das Fragment B enthält die Nukleotide 34 - 1230 des transkribierten Bereiches eines Patatin-Gens aus der Kartoffel (Rosahl *et al.,* vide supra), welche mittels PCR unter Verwendung des und des amplifiziert wurden. Die Primer beinhalten zusätzlich folgende Restriktionschnittstellen: 5'-Primer, Asp718; 3'-Primer, BamHI. Als Template diente der Klon pgT5 (Rosahl *et al.,* vide supra). Das resultierende Fragment wurde über Asp718 bzw. BamHI zwischen den 35S-Promotor und die Polyadenylierungsstelle des Octopin-Synthase-Gens eingefügt.
Das Fragment C enthält das Saccharoseisomerase-Gen aus *Erwinia rhapontici,* welches die Nukleotide 109 - 1803 umfaßt. Dieses ist über die BamHI-Schnittstelle am 5'-Ende mit Fragment B fusioniert, wobei ein durchgehendes Leseraster zwischen beiden Fragmenten erzeugt wird. Dadurch wird ein für die Aufnahme des Proteins in die Vakuole notwendiges Signalpeptid N-terminal an die Saccharoseisomerase-Sequenz fusioniert.
Das Fragment D enthält das Polyadenylierungssignal des Gens 3 der T-DNA des Ti-Plasmids pTiACH5 (Gielen *et al.,* vide supra), Nukleotide 11749 - 11939.

In p35S-vaclso (35S = 355-Promotor, vac = Vakuole, Iso = Saccharoseisomerase) steht die kodierende Region der Saccharoseisomerase aus *E. rhapontici* unter konstitutiver Kontrolle, das Genprodukt wird in die Vakuole aufgenommen.

### Beispiel 6

### Herstellung von Plasmid pB33-cwIso

In analoger Weise wie in Beispiel 4 beschrieben, jedoch unter Austausch des 35S-Promotors gegen den Promotor des Klasse I Patatin-Gens B33 (Rocha-Sosa *et al.* (1989) EMBO J. 8:23-29), wurde das Plasmid pB33-cwIso hergestellt. Die Expressionskassette dieses Plasmids besteht aus den drei Fragmenten A, B und C (siehe Abbildung 3).
Fragment A beinhaltet die Region -1512 bis +14 relativ zur Transkriptionsinitiationsstelle des Klasse I Patatin-Gens. Die Promotorregion wurde als DraI-Fragment in den mit SstI geschnittenen Vektor pUC18, dessen Enden unter Einsatz der T4-DNA-Polymerase aufgefüllt und somit geglättet worden waren, ligiert.
Das Fragment B enthält die Nukleotide 923 bis 1059 eines Proteinase-Inhibitor II-Gens aus der Kartoffel, welche über einen Linker mit der Sequenz ACC GAA TTG GG an das Saccharoseisomerase-Gen aus *E. rhapontici,* welches die Nukleotide, 109 bis 1803 umfasst, fusioniert sind. Dadurch wird ein für die Aufnahme von Proteinen in das ER notwendiges Signalpeptid eines pflanzlichen Proteins N-terminal an die Saccharoseisomerase-Sequenz fusioniert. Das Fragment B wurde als Asp718/SaII-Fragment aus dem oben beschriebenen Konstrukt p35S-cwIso (Beispiel 4) herausgeschnitten und zwischen die Restriktionsschnitistellen Asp718 und SaII der Polylinkerregion von pUC 18 kloniert.
Das Fragment C enthält das Polyadenylierungssignal des Gens 3 der T-DNA des Ti-Plasmids pTiACH5 (Gielen *et al.* (1984) EMBO J. 3:835), Nukleotide 11749 - 11939, welches als PvuII/HindIII-Fragment aus dem Plasmid pAGV 40 (Herrera-Estrella *et al.* (1983) Nature 303:209) isoliert und nach Addition von SphI-Linkern an die PvuII-Schnittstelle zwischen die SphI- und HindIII-Schnittstelle des Polylinkers von pUC 18 kloniert worden ist.

Das chimäre Gen wurde anschließend als EcoRI/HindIII-Fragment zwischen die EcoRI- und HindIII-Schnittstelle des Plasmids pBIN19 (Bevan (1984) Nucl. Acids Res. 12:8711) kloniert.

In pB33-cwIso (B33 = Promotor des Klasse I Patatin-Gens B33, cw = Zellwand, Iso = Saccharoseisomerase) steht die kodierende Region der Saccharoseisomerase aus *E. rhapontici* unter gewebespezifischer Kontrolle, das Genprodukt wird in das ER aufgenommen.

### Beispiel 7

### Herstellung von Plasmid pB33-vacIso

In analoger Weise wie in Beispiel 5 beschrieben, jedoch unter Austausch des 35S-Promotors gegen den Promotor des Klasse I Patatin-Gens B33 (Rocha-Sosa *et al.* (1989) EMBO J. 8:23-29), wurde das Plasmid pB33-vacIso hergestellt. Die Expressionskassette dieses Plasmids besteht aus den drei Fragmenten A, B und C (siehe Abbildung 3).
Fragment A beinhaltet die Region -1512 bis +14 relativ zur Transkriptionsinitiationsstelle des Klasse I Patatin-Gens. Die Promotorregion wurde als DraI-Fragment in den mit SstI geschnittenen Vektor pUC 18, dessen Enden unter Einsatz der T4-DNA-Polymerase aufgefüllt und somit geglättet worden waren, ligiert.
Das Fragment B enthält die Nukleotide 34-1230 des transkribierten Bereiches eines Patatin-Gens aus der Kartoffel (Rosahl *et al.,* vide supra), welche mit den Nukleotiden 109 - 1803 der kodierenden Region des Saccharoseisomerase translational fusioniert sind. Fragment B wurde als Asp718/SaII-Fragment aus dem Plasmid p35S-vacIso herausgeschnitten und zwischen die entsprechenden Restriktionsschnittstellen des Vektors pUC18 ligiert.
Das Fragment C enthält das Polyadenylierungssignal des Gens 3 der T-DNA des Ti-Plasmids pTiACH5 (Gielen *et al.* (1984) EMBO J. 3:835), Nukleotide 11749 - 11939, welches als PvuII/HindIII-Fragment aus dem Plasmid pAGV 40 (Herrera-Estrella *et al.* (1983) Nature 303:209) isoliert und nach Addition von SphI-Linkern an die PvuII-Schnittstelle zwischen die SphI- und HindIII-Schnittstelle des Polylinkers von pUC 18 kloniert worden ist.
Das chimäre Gen wurde anschließend als EcoRI/HindIII-Fragment zwischen die EcoRI- und HindIII-Schnittstelle des Plasmids pBIN19 (Bevan (1984) Nucl. Acids Res. 12:8711) kloniert.

In pB33-vacIso (B33 = Promotor des Klasse I Patatin-Gens B33, vac = Vakuole, Iso = Saccharoseisomerase) steht die kodierende Region der Saccharoseisomerase aus *E. rhapontici* unter gewebespezifischer Kontrolle. das Genprodukt wird in die Vakuole aufgenommen.

### Beispiel 8

### Herstellung des Plasmids pMAL-SucIso

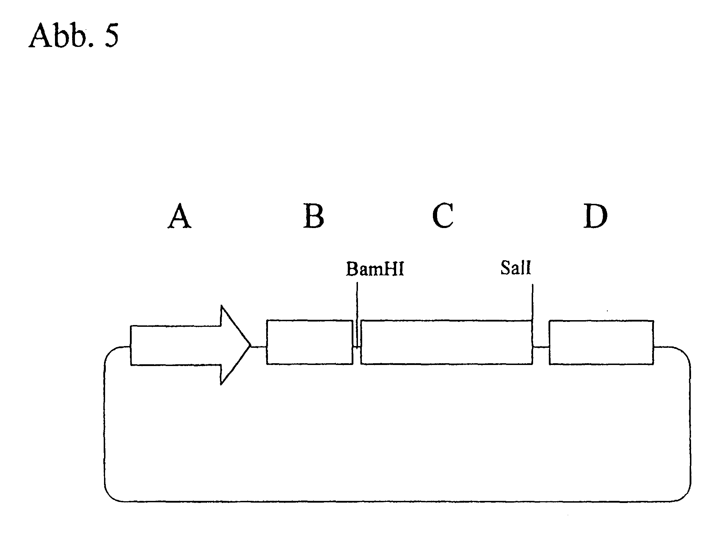

Zur Herstellung des Plasmids pMAL-Suciso wurde das Saccharoseisomerase-Fragment aus dem Konstrukt pCR-SucIso2 über die Restriktionsenzyme BamHI und SaII herausgeschnitten und in einen ebenfalls so geschnittenen pMAL-c2 Vektor (New England Biolabs) ligiert. wodurch das Konstrukt pMAL-SucIso (Abbildung 5) erhalten wurde. Dieses ermöglicht eine Expression des Enzyms als Fusionsprotein mit dem Maltose-bindenden Protein unter der Kontrolle des durch IPTG induzierbaren tac-Promotors.
Fragment A beinhaltet den tac-Promotor, der eine durch IPTG induzierbare Genexpression ermöglicht.
Fragment B beinhaltet einen Teil des malE-Gens mit Translationsstart.
Fragment C beinhaltet die kodierende Region der Saccharoseisomerase.
Fragment D beinhaltet den *rrnB*-Terminator aus *E. coli.*

Mit pMAL-Suciso transformierte Bakterienzellen zeigen IPTG-induzierbare Expression der Saccharoseisomerase aus *E. rhapontici.*

### Beispiel 9

### Funktioneller Nachweis der Saccharoseisomeraseaktivität in E. coli

Die funktionelle Charakterisierung des Saccharoseisomerase-Gens erfolgte durch Expression in *E.coli.* Dazu wurde das Plasmid pMAL-SucIso in *E. coli* (XL-I blue, Stratagene) transformiert. Die Expression des Fusionsproteins zwischen dem Maltose-bindenden Protein und der Saccharoseisomerase erfolgte nach Herstellerangaben in einem Kulturmaßstab von 50 ml. Nach Ernte der Zellen wurde das Pellet in 1 ml 50 mM Natriumphosphatpuffer (pH 6,0) resuspendiert und die lösliche Proteinfraktion durch Ultraschallbehandlung freigesetzt. Ein Aliquot des Rohextraktes wurden mit dem gleichen Volumen 600 mM Saccharose gemischt und für 24 Stunden bei 30 °C inkubiert. Zum Nachweis der entstandenen Palatinose wurde ein Aliquot des Ansatzes einer HPLC-Analyse unterzogen. Das Chromatogramm bestätigte die Produktion von Palatinose durch die rekombinante Saccharoseisomerase in *E. coli.*

### Beispiel 10

### In vivo-Nachweis der Saccharoseisomeraseaktivität in transgenen Pflanzen

Der Nachweis der in vivo-Funktionalität der Saccharoseisomerase in transgenen Pflanzen wurde wie folgt durchgeführt: von 0,5 cm² großen Blattscheiben von untransformierten Tabakpflanzen und den Transformanden 35S-cytIso und 35S-cwIso wurden ethanolische Extrakte hergestellt und durch HPLC analysiert und die Zucker anhand der entsprechenden Standards identifiziert. Wie die Chromatogramme zeigten. führte die Expression der Saccharoseisomerase in der Zellwand zu einer substantiellen Akkumulation von Palatinose in den untersuchten p35S-cwIso-Pflanzen. Dagegen konnte in den analysierten p35S-cytIso-Pflanzen, in denen die Saccharoseisomerase im Cytosol exprimiert wird, keine Enzymaktivität detektiert werden. Der Wildtyp enthält keine Palatinose, wie ebenfalls aus den Chromatogrammen deutlich zu erkennen war.

Die ebenfalls mittels HPLC-Analyse untersuchten transgenen Tabakpflanzen. die das Konstrukt p35S-vacIso enthielten und daher die Saccharoseisomerase in der Vakuole exprimierten, zeigten ebenfalls nachweisbare Akkumulation von Palatinose.

Der Gehalt an löslichen Zuckern in transgenen Tabakpflanzen, die das Konstrukt p35S-cwIso enthielten, ist in der folgenden Tabelle 1 dargestellt.

**Tabelle 1**

| | Glukose [mmol/m²] | Fruktose [mmol/m²] | Saccharose [mmol/m²] | Palatinose [mmol/m²] |
|---|---|---|---|---|
| Kontrolle | 0,27 | 0,13 | 0,03 | nicht detektierbar |
| p35S-cwIso 54 | 0,25 | 0,15 | 0,03 | 0,6 |
| p35S-cwlso 27 | 0,3 | 0.12 | 0,02 | 0,94 |
| p35S-cwIso 45 | 0,4 | 0,27 | 0,04 | 0,86 |

Wie oben beschrieben, wurden die Konstrukte pB33-cwIso und pB33-vacIso auf Kartoffel übertragen. Die analysierten Transformanten, in denen die Saccharoseisomerase-DNA-Sequenzen unter Kontrolle des Patatin B33-Promotors steht, zeigten in den Knollen nachweisbare Akkumulation von Palatinose.

Der Gehalt an löslichen Zuckern in transgenen Kartoffelknollen, die das Konstrukt pB33-cwIso enthielten, ist in der folgenden Tabelle 2 dargestellt (in µmol pro Gramm Frischgewicht).

**Tabelle 2**

| | Glukose [µmol/gFW] | Fruktose [µmol/gFW] | Saccharose [µmol/gFW] | Palatinose [µmol/gFW] |
|---|---|---|---|---|
| Kontrolle | 4,97 | 0,09 | 17,7 | nicht detektierbar |
| pB33-cwIso 5 | 0,56 | 0,55 | 2,6 | 70,5 |
| pB33-cwIso 17 | 0.84 | 2,53 | 1,5 | 81,3 |
| pB33-cwIso 23 | 0,45 | 0,27 | 7,6 | 89.6 |

Die in den transgenen Pflanzen produzierte Palatinose kann ähnlich wie konventionell hergestellte Palatinose durch Kristallisation aus dem Pflanzensaft isoliert werden.

## Patentansprüche

1. Rekombinantes Nukleinsäuremolekül, umfassend
a) regulatorische Sequenzen eines in Pflanzenzellen aktiven Promotors;
b) operativ daran gebunden eine DNA-Sequenz, die ein Protein mit der enzymatischen Aktivität einer Saccharoseisomerase kodiert und durch Signalsequenzen ergänzt ist, die den Transport des Proteins mit der enzymatischen Aktivität einer Saccharoseisomerase zu einem bestimmten Zellkompartiment oder einer bestimmten Zellorganelle gewährleisten; und
c) operativ daran gebunden regulatorische Sequenzen, die als Transkriptions-, Terminationsund/oder Polyadenylierungssignale in Pflanzenzellen dienen können.

2. Rekombinantes Nukleinsäuremolekül nach Anspruch 1, wobei die DNA-Sequenz ausgewählt ist aus der Gruppe bestehend aus
a) DNA-Sequenzen, die eine Nukleotidsequenz umfassen, die die in SEQ ID NO. 3 angegebene Aminosäuresequenz oder Fragmente davon kodiert,
b) DNA-Sequenzen, die die in SEQ ID No. 1 angegebene Nukleotidsequenz umfassen,
c) DNA-Sequenzen, die mit SEQ ID No. 1 in mindestens 60%, bevorzugt über 80% und besonders bevorzugt über 90% der Nukleotidsequenz übereinstimmen.

3. Rekombinantes Nukleinsäuremolekül nach Anspruch 1 oder 2, wobei der Promotor ein gewebe- oder organspezifischer oder ein induzierbarer Promotor ist.

4. Rekombinantes Nukleinsäuremolekül nach Anspruch 3, wobei der gewebe- oder organspezifische Promotor ein Speicherorgan-, Samen- oder Frucht-spezifischer Promotor ist.

5. Rekombinantes Nukleinsäuremolekül nach Anspruch 3, wobei der induzierbare Promotor ein durch chemische Verbindungen induzierbarer Promotor ist.

6. Rekombinantes Nukleinsäuremolekül nach einem der vorangehenden Ansprüche, wobei das Zellkompartiment oder die Zellorganelle die Zellwand bzw. der Apoplast ist.

7. Rekombinantes Nukleinsäuremolekül nach einem der Ansprüche 1 bis 5, wobei das Zellkompartiment oder die Zellorganelle die Vakuole oder das Endomembransystem ist.

8. Vektor, umfassend ein rekombinantes Nukleinsäuremolekül nach einem der vorangehenden Ansprüche.

9. Wirtszelle, enthaltend ein rekombinantes Nukleinsäuremolekül oder einen Vektor nach einem der vorangehenden Ansprüche.

10. Verfahren zur Produktion nicht-kariogener Zucker in transformierten Pflanzenzellen, umfassend die Schritte:
a) Herstellung eines rekombinanten Nukleinsäuremoleküls nach einem der Ansprüche 1 bis 7 oder eines Vektors nach Anspruch 8,
b) Übertragung des Nukleinsäuremoleküls oder des Vektors aus a) auf Pflanzenzellen.

11. Verfahren nach Anspruch 10, weiter umfassend in Schritt c) die Regeneration transgener Pflanzen aus den Pflanzenzellen.

12. Transgene Pflanzenzellen, enthaltend ein rekombinantes Nukleinsäuremolekül nach einem der Ansprüche 1 bis 7 oder einen Vektor nach Anspruche 8 oder hergestellt nach einem Verfahren nach Anspruch 10.

13. Transgene Pflanzen, enthaltend eine Pflanzenzelle nach Anspruch 12 oder hergestellt nach Anspruch 10, sowie Teile dieser Pflanzen, transgene Ernteprodukte und transgenes Vermehrungsmaterial dieser Pflanzen, wie Protoplasten, Pflanzenzellen, Kalli, Samen, Knollen, Stecklinge, sowie die transgenen Nachkommen dieser Pflanzen.

14. Verfahren zur Gewinnung nicht-kariogener Zucker aus transformierten Pflanzenzellen bzw. Pflanzen, umfassend die Schritte
a) Übertragung eines rekombinanten Nukleinsäuremoleküls nach einem der Ansprüche der 1 bis 7 oder eines Vektors nach Anspruch 8 auf Pflanzenzellen bzw. Pflanzen, und
b) Reinigen der nicht-kariogenen Zucker, insbesondere Palatinose, aus den Pflanzenzellen bzw. Pflanzen.

15. Verfahren nach Anspruch 14, wobei in Schritt b) die Reinigung durch Kristallisation erfolgt.

## Claims

1. A recombinant nucleic acid molecule comprising
a) regulatory sequences of a promoter which is active in plant cells,
b) operatively linked thereto a DNA sequence which codes for a protein having the enzymatic activity of a saccharose isomerase and to which signal sequences are added, which allow for the transport of the protein having the enzymatic activity of a saccharose isomerase to a specific cell compartment or a specific cell organelle, and
c) operatively linked thereto regulatory sequences, which can serve as transcription, termination and/or polyadenylation signals in plant cells.

2. The recombinant nucleic acid molecule according to claim 1, wherein the DNA sequence is selected from the group consisting of
a) DNA sequences comprising a nucleotide sequence that codes for the amino acid sequence given in SED ID NO. 3 or fragments thereof,
b) DNA sequences comprising the nucleotide sequence given in SEQ ID NO. 1,
c) DNA sequences which correspond to SEQ ID NO. 1 in at least 60%, preferably above 80% and especially preferably above 90% of the nucleotide sequence.

3. The recombinant nucleic acid molecule according to claim 1 or 2, wherein the promoter is a tissue- or organ-specific promoter or an inducible promoter.

4. The recombinant nucleic acid molecule according to claim 3, wherein the tissue- or organ-specific promoter is a storage organ-, seed- or fruit-specific promoter.

5. The recombinant nucleic acid molecule according to claim 3, wherein the inducible promoter is inducible by chemical compounds.

6. The recombinant nucleic acid molecule according to any of the preceding claims, wherein the cell compartment or the cell organelle is the cell wall or the apoplast.

7. The recombinant nucleic acid molecule according to any of the claims 1 to 5, wherein the cell compartment or the cell organelle is the vacuole or the endomembrane system.

8. A vector comprising a recombinant nucleic acid molecule according to any of the preceding claims.

9. A host cell containing a recombinant nucleic acid molecule or containing a vector according to any of the preceding claims.

10. A method for the production of non-cariogenic sugars in transformed plant cells, comprising the steps:
a) producing a recombinant nucleic acid molecule according to any of the claims 1 to 7 or a vector according to claim 8,
b) transferring the nucleic acid molecule or the vector from a) to plant cells.

11. The method according to claim 10, further comprising in step c) regenerating transgenic plants from the plant cells.

12. Transgenic plant cells containing a recombinant nucleic acid molecule according to any of the claims 1 to 7 or a vector according to claim 8 or produced by a method according to claim 10.

13. Transgenic plants containing a plant cell according to claim 12, or produced by a method according to claim 10, as well as parts of these plants, transgenic harvest products and transgenic propagating material of these plants, such as protoplasts, plant cells, calli, seeds, tubers, cuttings, and the transgenic progeny of these plants.

14. A method for obtaining non-cariogenic sugars from transformed plant cells or plants, comprising the steps:
a) transferring a recombinant nucleic acid molecule according to any of the claims 1 to 7 or a vector according to claim 8 to plant cells or plants, and
b) purifying the non-cariogenic sugars, especially palatinose, from these plant cells or plants.

15. The method according to claim 14, wherein purifying in step b) is carried out by crystallization.

## Revendications

1. Molécule d'acide nucléique recombinante comprenant
a) des séquences régulatrices d'un promoteur actif dans des cellules végétales ;
b) liée à ces dernières de façon opérationnelle, une séquence ADN, codante pour une protéine avec l'activité enzymatique d'une saccharose isomérase et qui est complétée par des séquences de signaux, assurant le transport de la protéine avec l'activité enzymatique d'une saccharose isomérase vers un compartiment cellulaire défini ou un organule cellulaire défini ; et
c) des séquences régulatrices qui y sont liées de façon opérationnelle, pouvant servir de signaux de transcription, de terminaison et/ou de polyadénylation dans des cellules végétales.

2. Molécule d'acide nucléique recombinante selon la revendication 1, la séquence ADN étant choisie dans le groupe constitué
a) de séquences ADN, comprenant une séquence nucléotide, codante pour la séquence d'acide aminé ou des fragments mentionnés dans SEQ ID NO. 3
b) de séquences ADN, comprenant la séquence nucléotide mentionnée dans SEQ ID No. 1,
c) de séquences ADN correspondant avec SEQ ID. No. 1 à au moins 60 %, de préférence à plus de 80 % et de façon notamment préconisée à plus de 90 % de la séquence nucléotide.

3. Molécule d'acide nucléique recombinante selon l'une quelconque des revendications 1 ou 2, le promoteur étant un promoteur spécifique aux tissus ou aux organes ou un promoteur inductible.

4. Molécule d'acide nucléique recombinante selon la revendication 3, le promoteur spécifique aux tissus ou aux organes étant un promoteur spécifique aux organes de stockage, aux semences ou aux fruits.

5. Molécule d'acide nucléique recombinante selon la revendication 3, le promoteur inductible étant un promoteur inductible par composés chimiques.

6. Molécule d'acide nucléique recombinante selon l'une quelconque des revendications précédentes, le compartiment cellulaire ou l'organule cellulaire étant une membrane cellulaire ou l'apoplaste.

7. Molécule d'acide nucléique recombinante selon l'une quelconque des revendications 1 à 5, le compartiment cellulaire ou l'organule cellulaire étant la vacuole ou le système d'endomembrane.

8. Vecteur, comprenant une molécule d'acide nucléique recombinante selon l'une quelconque des revendications précédentes.

9. Cellule hôte, contenant une molécule d'acide nucléique recombinante ou un vecteur selon l'une quelconque des revendications précédentes.

10. Procédé pour la production de sucre non cariogène, en cellules végétales transformées, comprenant les étapes suivantes :
a) Fabrication d'une molécule d'acide nucléique recombinante, selon l'une quelconque des revendications 1 à 7 ou d'un vecteur selon la revendication 8,
b) Transfert de la molécule d'acide nucléique ou du vecteur issu de a) sur des cellules végétales.

11. Procédé selon la revendication 10, comprenant en plus sous l'étape c) la régénération de plantes transgéniques issus des cellules végétales.

12. Cellules végétales transgéniques, contenant une molécule d'acide nucléique recombinante selon l'une quelconque des revendications 1 à 7 ou un vecteur selon la revendication 8 ou fabriqué selon un procédé selon la revendication 10.

13. Plantes transgéniques contenant une cellule végétale selon la revendication 12 ou fabriqués selon la revendication 10, ainsi que parties de ces plantes, produits de récolte transgéniques et matières de multiplication de ces plantes, comme des protoplastes, des cellules végétales, calli, semences, tubercules, boutures et descendants transgéniques de ces plantes.

14. Procédé d'extraction de sucre non cariogène, à partir de cellules végétales ou de plantes transformés, comprenant les étapes suivantes :
a) transfert d'une molécule d'acide nucléique recombinante selon l'une quelconque des revendications 1 à 7 ou d'un vecteur selon la revendication 8 sur des cellules végétales ou des végétaux et
b) purification des sucres non cariogènes, notamment de palatinose, à partir des cellules végétales ou des végétaux.

15. Procédé selon la revendication 14, la purification au cours de l'étape b) ayant lieu par cristallisation.
